(19) Europäisches Patentamt
European Patent Office
Office européen des brevets

(11) Veröffentlichungsnummer: **0 298 034**
**A2**

(12) # EUROPÄISCHE PATENTANMELDUNG

(21) Anmeldenummer: **88810430.4**

(22) Anmeldetag: **22.06.88**

(51) Int. Cl.[4]: **C 07 C 50/16**
C 07 C 46/00, C 08 K 5/08,
C 08 L 63/00

(30) Priorität: **01.07.87 GB 8715436**

(43) Veröffentlichungstag der Anmeldung:
**04.01.89 Patentblatt 89/01**

(84) Benannte Vertragsstaaten:
**CH DE FR GB LI NL**

(71) Anmelder: **CIBA-GEIGY AG**
**Klybeckstrasse 141**
**CH-4002 Basel (CH)**

(72) Erfinder: **Howell, Frederick Harold, Dr.**
**27 High Bank**
**Atherton Manchester M29 9HZ (GB)**

**Duthaler, Rudolf, Dr.**
**Girenhaldenweg 17**
**CH-4126 Bettingen (CH)**

**Finter, Jürgen, Dr.**
**Zasiusstrasse 100**
**D-7800 Freiburg (DE)**

**Oertle, Konrad, Dr.**
**Vogesenstrasse 10**
**CH-4106 Therwil (CH)**

**Ramanathan, Visvanathan, Dr.**
**Friedensgasse 24**
**CH-4056 Basel (CH)**

(54) **Substituierte Anthrachinone und deren Verwendung in Epoxidharzen.**

(57) Es werden Anthrachinone mit funktionell substituierten, z.B. -COOH oder $-CONH-NH_2$, α, α-(Dialkyl)alkylgruppen beschrieben. Zusammensetzungen, die solche Anthrachinone, einen Aminoalkohol, ein Epoxidharz und gegebenenfalls einen Härter enthalten, eignen sich zur Herstellung metallischer Ueberzüge und Abbildungen auf der gehärteten Zusammensetzung durch Belichtung und stromlose Metallabscheidung.

EP 0 298 034 A2

## Beschreibung

### Substituierte Anthrachinone und deren Verwendung in Epoxidharzen

Die vorliegende Erfindung betrifft Anthrachinone mit funktionell substituierten α,α-(Dialkyl)alkylgruppen; Zusammensetzungen, die a) solche Anthrachinone, b) einen Aminoalkyl, c) ein Epoxidharz mit durchschnittlich mehr als einer Epoxidgruppe im Molekül, und d) gegebenenfalls einen Härter enthalten; sowie die Verwendung der gehärteten Zusammensetzung zur Herstellung von metallischen Ueberzügen oder Bildern.

In der EP-A-0 112 798 sind lichtempfindliche, vernetzte Reaktionsprodukte auf der Basis von Epoxidharzen beschrieben. Unter Mitverwendung von Metallsalzen der Gruppen Ib und VIII des Periodensystems der Elemente können durch Belichtung Metallkeime erzeugt werden, die man durch stromlose Metallabscheidung verstärken kann. Es ist wünschenswert, auf lichtempfindlichen Epoxidharzen direkt und ohne Mitverwendung von Metallsalzen metallische Ueberzüge oder Abbildungen durch stromlose Metallabscheidung zu erzeugen.

Ein Gegenstand der Erfindung sind Verbindungen der Formel I

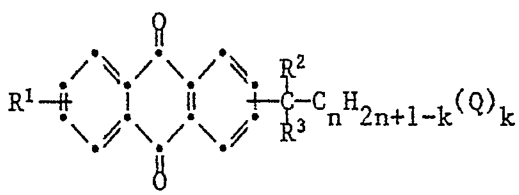

(I),

worin

n eine Zahl von 1 bis 17 darstellt,

k für 1 oder 2 steht und Q -COOH oder -CONR$^4$-NH$_2$ bedeutet, oder k für 1 steht und Q -OH, -NHR$^4$, -OCOR$^5$, -N(R$^4$)COR$^5$ oder

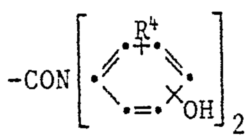

darstellt, worin R$^4$ H oder C$_1$-C$_4$-Alkyl ist und R$^5$ den um eine Carboxylgruppe verminderten Rest einer Tricarbonsäure oder Tetracarbonsäure bedeutet, R$^2$ und R$^3$ unabhängig voneinander lineares oder verzweigtes C$_1$-C$_5$-Alkyl darstellen, das unsubstituiert ist oder durch -COOH oder -CONR$^4$-NH$_2$ substituiert ist, falls Q -COOH oder -CONR$^4$-NH$_2$ darstellt, oder R$^2$ zusammen mit der $C_nH_{2n+1-k}$-Gruppe C$_5$-C$_{12}$-Cycloalkylen bildet und R$^3$ für C$_1$-C$_5$-Alkyl steht; und R$^1$ H, lineares oder verzweigtes C$_1$-C$_8$-Alkyl, unsubstituiertes oder mit ein oder zwei Halogen substituiertes C$_7$-C$_9$-Aralkyl, unsubstituiertes oder mit C$_1$-C$_4$-Alkyl substituiertes C$_5$-C$_{12}$-Cycloalkyl bedeutet oder einen Rest der Formel $-CR^2R^3-C_nH_{2n+1-k}(Q)_k$ darstellt.

In Formel I bedeutet n bevorzugt eine Zahl von 1 bis 12, besonders von 1 bis 8 und insbesondere von 1 bis 5, und k ist bevorzugt 1. In einer bevorzugten Untergruppe sind n eine Zahl von 1 bis 8 und k = 1.

Q steht bevorzugt für -COOH und besonders für -CO-NR$^4$-NH$_2$, worin R$^4$ H oder Methyl ist. R$^5$ ist bevorzugt der Rest von Trimellitsäure oder Pyromellitsäure. Im Rest

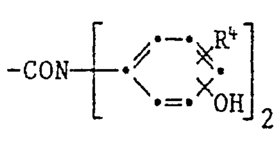

ist die -OH-Gruppe bevorzugt in p-Stellung gebunden und R$^4$ bedeutet bevorzugt H oder Methyl.

Die Gruppe $-CR^2R^3C_nH_{2n+1-k}(Q)_k$ ist bevorzugt in 6- oder 7-Stellung. Der Rest R$^1$ stellt für diesen Fall besonders die Gruppe $-CR^2R^3C_nH_{2n+1-k}(Q)_k$ dar.

R$^1$ stellt bevorzugt H oder den Rest der Formel $-CR^2R^3C_nH_{2n+1-k}(Q)_k$ dar. In der Bedeutung von Alkyl enthält R$^1$ bevorzugt 1 bis 4 C-Atome. Beispiele für R$^1$ als Alkyl sind Methyl, Ethyl, n- und i-Propyl, n-, i- und t-Butyl, Pentyl, Hexyl, 2-Ethylhexyl, Heptyl und Octyl. R$^1$ als gegebenenfalls mit Halogen substituiertes Aralkyl kann z.B. 1-Phenylprop-1-yl, 1-Phenylprop-2-yl, 1-Phenylprop-3-yl, 1-Phenyleth-1-yl, 1-Phenyleth-2-yl, p-Chlorbenzyl und besonders Benzyl sein. R$^1$ enthält als Cycloalkyl bevorzugt 5 oder 6 Ring-C-Atome; Beispiele für R$^1$

gegebenenfalls mit Alkyl substituiertes Cycloalkyl sind Cyclopentyl, Methylcyclohexyl, Methylcyclopentyl, Cyclohexyl, Dimethylcyclohexyl, Butylcyclohexyl.

$R^2$ und $R^3$ sind bevorzugt unabhängig voneinander $C_1$-$C_3$-Alkyl und insbesondere Methyl oder Ethyl. $R^2$ und $R^3$ sind bevorzugt lineares Alkyl. $R^2$ kann zusammen mit der $C_nH_{2n-k}$-Gruppe Cycloalkylen mit bevorzugt 5 oder 6 Ring-C-Atomen bilden, wobei $R^3$ $C_1$-$C_5$-, vorzugsweise $C_1$-$C_3$-Alkyl bedeutet.

$R^4$ in der Bedeutung von Alkyl kann z.B. Butyl, Propyl, Ethyl oder Methyl sein. Bevorzugt steht $R^4$ für H oder Methyl.

Eine bevorzugte Untergruppe von Verbindungen der Formel I sind jene, worin in Formel I $R^1$ für H oder einen Rest der Formel $-CR^2R^3C_nH_{2n+1-k}(Q)_k$ steht, $R^2$ und $R^3$ unabhängig voneinander Methyl oder Ethyl bedeuten, n für eine Zahl von 1 bis 8 und k für 1 stehen, Q -COOH oder $-CONR^4-NH_2$ darstellt, und $R^4$ H oder $C_1$-$C_4$-Alkyl bedeutet.

Ein weiterer Gegenstand der Erfindung sind Verbindungen der Formel Ia

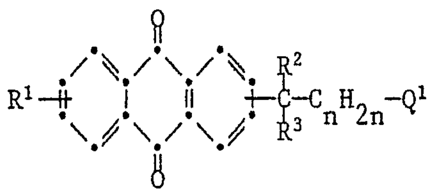 (Ia)

worin

$R^1$ H, lineares oder verzweigtes $C_1$-$C_8$-Alkyl, unsubstituiertes oder mit 1 oder 2 Halogen substituiertes $C_7$-$C_9$-Aralkyl, unsubstituiertes oder mit $C_1$-$C_4$-Alkyl substituiertes $C_5$-$C_{12}$-Cycloalkyl oder einen Rest der Formel $-CR^2R^3-C_nH_{2n}-Q^1$ darstellt; $R^2$ und $R^3$ unabhängig voneinander lineares oder verzweigtes $C_1$-$C_5$-Alkyl darstellen, oder $R^2$ zusammen mit der $C_nH_{2n}$-Gruppe $C_5$-$C_{12}$-Cycloalkylen bildet und $R^3$ H oder $C_1$-$C_5$-Alkyl ist; n für eine Zahl von 1 bis 17 steht; und $Q^1$ für -CN, -COCl, $-CONHR^4$, $-CON(R^4)_2$, $-COR^6$, $-O-C_1-C_8$-Acyl oder $-NR^4-C_1-C_8$-Acyl steht, worin $R^4$ H oder $C_1$-$C_4$-Alkyl bedeutet und $R^6$ der Rest eines einwertigen Alkohols ist. Für $R^1$ bis $R^4$, n und k gelten die gleichen Bevorzugungen wie für die Verbindungen der Formel I. $R^6$ als Rest eines einwertigen Alkohols enthält bevorzugt 1 bis 12, besonders 1 bis 6 C-Atome. Beispiel für solche Reste sind Methoxy, Ethoxy, Propoxy, Butoxy, Pentoxy, Hexoxy, Phenoxy, Benzyloxy, Octoxy, Decyloxy und Dodecyloxy. Das Acyl kann 1 bis 8, vorzugsweise 1 bis 4 C-Atome enthalten. Beispiele für Acyl sind Formyl, Acetyl, Propionyl, Butanoyl, Pentanoyl, Hexanoyl und Benzoyl. Die Verbindungen der Formel Ia sind Zwischenprodukte zur Herstellung von Verbindungen der Formel I durch eine übliche Derivatisierung.

Beispiele für Verbindungen der Formeln I und Ia sind:

2-(3′-Carboxy-2′-methyl-pròp-2′-yl)-9,10-anthrachinon,
2-(3′-Methoxycarbonyl-2′-methyl-prop-2′-yl)-9,10-anthrachinon,
2-(3′-Ethoxycarbonyl-2′-methyl-prop-2′-yl)-9,10-anthrachinon,
2-(3′-Propoxycarbonyl-2′-methyl-prop-2′-yl)-9,10-anthrachinon,
2-(3′-iso-Propoxycarbonyl-2′-methyl-prop-2′-yl)-9,10-anthrachinon,
2-(3′-Butoxycabronyl-2′-methyl-prop-2′-yl)-9,10-anthrachinon,
2-(3′-Hexyloxycarbonyl-2′-methyl-prop-2′-yl)-9,10-anthrachinon,
2-[3′-(2″-Ethylhexyloxycarbonyl)-2′-methyl-prop-2′-yl]-9,10-anthrachinon,
2-(3′-Dodecyloxycarbonyl-2′-methyl-prop-2′-yl)-9,10-anthrachinon,
2-(3′-Octadecyloxycarbonyl-2′-methyl-prop-2′-yl)-9,10-anthrachinon,
2-(3′-Carbamoyl-2′-methyl-prop-2′-yl)-9,10-anthrachinon,
2-(3′-Carbohydrazido-2′-methyl-prop-2′-yl)-9,10-anthrachinon,
2-(4′-Hydroxy-2′-methyl-but-2′-yl)-9,10-anthrachinon,
2-(4′-Acetoxy-2′-methyl-but-2′-yl)-9,10-anthrachinon,
2-(4′-Amino-2′-methyl-but-2′-yl)-9,10-anthrachinon,
2-(4′-Acetamido-2′-methyl-but-2′-yl)-9,10-anthrachinon,
2-(4′-Carbohydrazido-2′-methyl-but-2′-yl)-9,10-anthrachinon,
2-(4′-Carboxy-2′-methyl-but-2′-yl)-9,10-anthrachinon,
2-(5′-Carboxy-2′-methyl-pent-2′-yl)-9,10-anthrachinon,
2-(5′-Methoxycarbonyl-2′-methyl-pent-2′-yl)-9,10-anthrachinon,
2-(5′-Ethoxycarbonyl-2′-methyl-pent-2′-yl)-9,10-anthrachinon
2-(5′-Propoxycarbonyl-2′-methyl-pent-2′-yl)-9,10-anthrachinon,,
2-(5′-iso-Propoxycarbonyl-2′-methyl-pent-2′-yl)-9,10-anthrachinon,
2-(5′-Butoxycarbonyl-2′-methyl-pent-2′-yl)-9,10-anthrachinon,
2-(5′-Hexyloxycarbonyl-2′-methyl-pent-2′-yl)-9,10-anthrachinon,
2-[5′-(2″-Ethylhexyloxycarbonyl)-2′-methyl-pent-2′-yl]-9,10-anthrachinon,
2-(5′-Dodecyloxycarbonyl-2′-methyl-pent-2′-yl)-9,10-anthrachinon,
2-(5′-Octadecyloxycarbonyl-2′-methyl-pent-2′-yl)-9,10-anthrachinon,

2-(5′-Cyclohexyloxycarbonyl-2′-methyl-pent-2′-yl)-9,10-anthrachinon,
2-(5′-n-Dec-10-enyloxycarbonyl-2′-methyl-pent-2′-yl)-9,10-anthrachinon,
2-(5′-Benzyloxycarbonyl-2′-methyl-pent-2′-yl)-9,10-anthrachinon,
2-(5′-Allyloxycarbonyl-2′-methyl-pent-2′-yl)-9,10-anthrachinon,
2-(5′-Carbamoyl-2′-methyl-pent-2′-yl)-9,10-anthrachinon,
2-(5′-N,N-Butylcarbamoyl-2′-methyl-pent-2′-yl)-9,10-anthrachinon,
2-(5′-N-Allylcarbamoyl-2′-methyl-pent-2′-yl)-9,10-anthrachinon,
2-(5′-N,N-Di-allylcarbamoyl-2′-methyl-pent-2′-yl)-9,10-anthrachinon,
2-(5′-N-Benzylcarbamoyl-2′-methyl-pent-2′-yl)-9,10-anthrachinon,
2-(5′-Cyano-2′-methyl-pent-2′-yl)-9,10-anthrachinon,
2-(5′-Hydroxy-2′-methyl-pent-2′-yl)-9,10-anthrachinon,
2-(6′-Amino-2′-methyl-hex-2′-yl)-9,10-anthrachinon,
2-(6′-Acetamido-2′-methyl-hex-2′-yl)-9,10,anthrachinon,
2-(6′-Amino-2′-methyl-hept-2′-yl)-9,10-anthrachinon,
2-(6′-Acetamido-2′-methyl-hept-2′-yl)-9,10-anthrachinon,
2-(6′-Hydroxy-2′-methyl-hex-2′-yl)-9,10-anthrachinon,
2-(6′-Methoxy-2′-methyl-hex-2′-yl)-9,10-anthrachinon,
2-(6′-Acetoxy-2′-methyl-hex-2′-yl)-9,10-anthrachinon,
2-(6′-Chlorcarbonyl-2′-methyl-hex-2′-yl)-9,10-anthrachinon,
2-(5′-Carboxy-2′-methyl-pent-2′-yl)-9,10-anthrachinon,
2-(5′-Carbohydrazido-2′-methyl-pent-2′-yl)-9,10-anthrachinon,
2-(6′-Cyano-2′-methyl-hex-2′-yl)-9,10-anthrachinon,
2-(6′-Carboxy-2′-methyl-hex-2′-yl)-9,10-anthrachinon,
2-(6′-Carbohydrazido-2′-methyl-hex-2′-yl)-9,10-anthrachinon,
2-(7′-Carboxy-2′,6′-dimethyl-hept-2′-yl)-9,10-anthrachinon,
2-(7′-Methoxycarbonyl-2′,6′-dimethyl-hept-2′-yl)-9,10-anthrachinon,
2-(7′-Ethoxycarbonyl-2′,6′-dimethyl-hept-2′-yl)-9,10-anthrachinon,
2-(7′-Propoxycarbonyl-2′,6′-dimethyl-hept-2′-yl)-9,10-anthrachinon,
2-(7′-iso-Propoxycarbonyl-2′,6′-dimethyl-hept-2′-yl)-9,10-anthrachinon,
2-(7′-Butoxycarbonyl-2′,6′-dimethyl-hept-2′-yl)-9,10-anthrachinon,
2-(7′-Hexyloxycarbonyl-2′,6′-dimethyl-hept-2′-yl)-9,10-anthrachinon,
2-[5′-(2″-Ethylhexyloxycarbonyl)-2′,6′-dimethyl-hept-2′-yl)-9,10-anthrachinon,
2-(5′-Dodecyloxycarbonyl-2′,6′-dimethyl-hept-2′-yl)-9,10-anthrachinon,
2-(5′-Octadecyloxycarbonyl-2′,6′-dimethyl-hept-2′-yl)-9,10-anthrachinon,
2-(7′-Carbamoyl-2′,6′-dimethyl-hept-2′-yl)-9,10-anthrachinon,
2-(8′-Amino-2′,6′-dimethyl-oct-2′-yl)-9,10-anthrachinon,
2-(8′-Acetamido-2′,6′-dimethyl-oct-2′-yl)-9,10-anthrachinon,
2-(7′-Cyano-2′,6′-dimethyl-hept-2′-yl)-9,10-anthrachinon,
2-(8′-Hydroxy-2′,6′-dimethyl-oct-2′-yl)-9,10-anthrachinon,
2-(8′-Acetoxy-2′,6′-dimethyl-oct-2′-yl)-9,10-anthrachinon,
2-(8′-Carboxy-2′,6′-dimethyl-oct-2′-yl)-9,10-anthrachinon,
2-(8′-n-Benzyloxycarbonyl-2′,6′-dimethyl-oct-2′-yl)-9,10-anthrachinon,
2-(8′-n-Methylamino-2′,6′-dimethyl-oct-2′-yl)-9,10-anthrachinon,
2-(8′-Chlorcarbonyl-2′,6′-dimethyl-oct-2′-yl)-9,10-anthrachinon,
2-(8′-Cyano-2′,6′-dimethyl-oct-2′-yl)-9,10-anthrachinon,
cis und trans 2-(4′-Carboxy-1′-methyl-cyclo-hex-1′-yl)9,10-anthrachinon,
cis und trans 2-(4′-Methoxycarbonyl-1′-methyl-cyclo-hex-1′-yl)-9,10-anthrachinon,
2,6-Bis-(3′-carboxy-2′-methyl-prop-2′-yl)-9,10-anthrachinon,
2,7-Bis-(3′-carboxy-2′-methyl-prop-2′-yl)-9,10-anthrachinon,
2,6-Bis-(3′-methoxycarbonyl-2′-methyl-prop-2′-yl)-9,10-anthrachinon,
2,7-Bis-(3′-methoxycarbonyl-2′-methyl-prop-2′-yl)-9,10-anthrachinon,
2,6-Bis-(3′-ethoxycarbonyl-2′-methyl-prop-2′-yl)-9,10-anthrachinon,
2,7-Bis-(3′-ethoxycarbonyl-2′-methyl-prop-2′-yl)-9,10-anthrachinon,
2,6-Bis-(3′-propoxycarbonyl-2′-methyl-prop-2′-yl)-9,10-anthrachinon,
2,7-Bis-(3′-propoxycarbonyl-2′-methyl-prop-2′-yl)-9,10-anthrachinon,
2,6-Bis-(3′-isopropoxycarbonyl-2′-methyl-prop-2′-yl)-9,10-anthrachinon,
2,7-Bis-(3′-isopropoxycarbonyl-2′-methyl-prop-2′-yl)-9,10-anthrachinon,
2,6-Bis-(3′-butoxycarbonyl-2′-methyl-prop-2′-yl)-9,10-anthrachinon,
2,7-Bis-(3′-butoxycarbonyl-2′-methyl-prop-2′-yl)-9,10-anthrachinon,
2,6-Bis-(3′-hexyloxycarbonyl-2′-methyl-prop-2′-yl)-9,10-anthrachinon,
2,7-Bis-(3′-hexyloxycarbonyl-2′-methyl-prop-2′-yl)-9,10-anthrachinon,
2,6-Bis-[3′-(2″-ethylhexyloxycarbonyl)-2′-methyl-prop-2′-yl]-9,10-anthrachinon,
2,7-Bis-[3′-(2″-ethylhexyloxycarbonyl)-2′-methyl-prop-2′-yl]-9,10-anthrachinon,
2,6-Bis-(3′-dodecyloxycarbonyl-2′-methyl-prop-2′-yl)-9,10-anthrachinon,
2,7-Bis-(3′-dodecyloxycarbonyl-2′-methyl-prop-2′-yl)-9,10-anthrachinon,

2,6-Bis-(3′-octadecyloxycarbonyl-2′-methyl-prop-2′-yl)-9,10-anthrachinon,
2,7-Bis-(3′-octadecyloxycarbonyl-2′-methyl-prop-2′-yl)-9,10-anthrachinon,
2,6-Bis-(4′-hydroxy-2′-methyl-but-2′-yl)-9,10-anthrachinon,
2,7-Bis-(4′-hydroxy-2′-methyl-but-2′-yl)-9,10-anthrachinon,
2,6-Bis-(4′-acetoxy-2′-methyl-but-2′-yl)-9,10-anthrachinon,
2,7-Bis-(4′-acetoxy-2′-methyl-but-2′-yl)-9,10-anthrachinon,
2,6-Bis-(5′-carboxy-2′-methyl-pent-2′-yl)-9,10-anthrachinon,
2,7-Bis-(5′-carboxy-2′-methyl-pent-2′-yl)-9,10-anthrachinon,
2,6-Bis-(5′-metoxycarbonyl-2′-methyl-pent-2′-yl)-9,10-anthrachinon,
2,7-Bis-(5′-metoxycarbonyl-2′-methyl-pent-2′-yl)-9,10-anthrachinon,
2,6-Bis-(5′-ethoxycarbonyl-2′-methyl-pent-2′-yl)-9,10-anthrachinon,
2,7-Bis-(5′-ethoxycarbonyl-2′-methyl-pent-2′-yl)-9,10-anthrachinon,
2,6-Bis-(5′-propoxycarbonyl-2′-methyl-pent-2′-yl)-9,10-anthrachinon,
2,7-Bis-(5′-propoxycarbonyl-2′-methyl-pent-2′-yl)-9,10-anthrachinon,
2,6-Bis-(5′-isopropoxycarbonyl-2′-methyl-pent-2′-yl)-9,10-anthrachinon,
2,7-Bis-(5′-isopropoxycarbonyl-2′-methyl-pent-2′-yl)-9,10-anthrachinon,
2,6-Bis-(5′-butoxycarbonyl-2′-methyl-pent-2′-yl)-9,10-anthrachinon,
2,7-Bis-(5′-butoxycarbonyl-2′-methyl-pent-2′-yl)-9,10-anthrachinon,
2,6-Bis-(5′-hexyloxycarbonyl-2′-methyl-pent-2′-yl)-9,10-anthrachinon,
2,7-Bis-(5′-hexyloxycarbonyl-2′-methyl-pent-2′-yl)-9,10-anthrachinon,
2,6-Bis-[5′-(2″-ethylhexyloxycarbonyl)-2′-methyl-pent-2′-yl)-9,10-anthrachinon,
2,7-Bis-[5′-(2″-ethylhexyloxycarbonyl)-2′-methyl-pent-2′-yl)-9,10-anthrachinon,
2,6-Bis-(5′-carbohydrazido-2′-methyl-pent-2′-yl)-9,10-anthrachinon,
2,7-Bis-(5′-carbohydrazido-2′-methyl-pent-2′-yl)-9,10-anthrachinon,
2,6-Bis-(5′-octadecyloxycarbonyl-2′-methyl-pent-2′-yl)-9,10-anthrachinon,
2,7-Bis-(5′-octadecyloxycarbonyl-2′-methyl-pent-2′-yl)-9,10-anthrachinon,
2,6-Bis-(5′-cyano-2′-methyl-pent-2′-yl)-9,10-anthrachinon,
2,7-Bis-(5′-cyano-2′-methyl-pent-2′-yl)-9,10-anthrachinon,
2,6-Bis-(6′-hydroxy-2′-methyl-hex-2′-yl)-9,10-anthrachinon,
2,7-Bis-(6′-hydroxy-2′-methyl-hex-2′-yl)-9,10-anthrachinon,
2,6-Bis-(6′-acetoxy-2′-methyl-hex-2′-yl)-9,10-anthrachinon,
2,7-Bis-(6′-acetoxy-2′-methyl-pent-2′-yl)-9,10-anthrachinon,
2,6-Bis-(5′-carbamoyl-2′-methyl-pent-2′-yl)-9,10-anthrachinon,
2,7-Bis-(5′-carbamoyl-2′-methyl-pent-2′-yl)-9,10-anthrachinon,
2,6-Bis-(5′-N-methylcarbamoyl-2′-methyl-pent-2′-yl)-9,10-anthrachinon,
2,7-Bis-(5′-N-methylcarbamoyl-2′-methyl-pent-2′-yl)-9,10-anthrachinon,
2,6-Bis-(5′-N,N-dimethylcarbamoyl-2′-methyl-pent-2′-yl)-9,10-anthrachinon,
2,7-Bis-(5′-N,N-dimethylcarbamoyl-2′-methyl-pent-2′-yl)-9,10-anthrachinon,
2,6-Bis-(5′-N-n-butylcarbamoyl-2′-methyl-pent-2′-yl)-9,10-anthrachinon,
2,7-Bis-(5′-N-n-butylcarbamoyl-2′-methyl-pent-2′-yl)-9,10-anthrachinon,
2,6-Bis-(6′-cyano-2′-methyl-hex-2′-yl)-9,10-anthrachinon,
2,7-Bis-(6′-carboxy-2′-methyl-hex-2′-yl)-9,10-anthrachinon,
2,6-Bis-(6′-chlorocarbonyl-2′-methyl-hex-2′-yl)-9,10-anthrachinon,
2,7-Bis-(6′-amino-2′-methyl-hex-2′-yl)-9,10-anthrachinon,
2,6-Bis-(6′-carbohydrazido-2′-methyl-hex-2′-yl)-9,10-anthrachinon,
2,6-Bis-(7′-carboxy-2′,6′-dimethyl-hept-2′-yl)-9,10-anthrachinon,
2,7-Bis-(7′-carboxy-2′,6′-dimethyl-hept-2′-yl)-9,10-anthrachinon,
2,6-Bis-(7′-methoxycarbonyl-2′,6′-dimethyl-hept-2′-yl)-9,10-anthrachinon,
2,7-Bis-(7′-methoxycarbonyl-2′,6′-dimethyl-hept-2′-yl)-9,10-anthrachinon,
2,6-Bis-(7′-cyano-2′,6′-dimethyl-hept-2′-yl)-9,10-anthrachinon,
2,7-Bis-(7′-cyano-2′,6′-dimethyl-hept-2′-yl)-9,10-anthrachinon,
2,6-Bis-(8′-hydroxy-2′,6′-dimethyl-oct-2′-yl)-9,10-anthrachinon,
2,7-Bis-(8′-hydroxy-2′,6′-dimethyl-oct-2′-yl)-9,10-anthrachinon,
2,6-Bis-(8′-acetoxy-2′,6′-dimethyl-oct-2′-yl)-9,10-anthrachinon,
2,7-Bis-(8′-acetoxy-2′,6′-dimethyl-oct-2′-yl)-9,10-anthrachinon,
2,6-Bis-(8′-cyano-2′,6′-dimethyl-oct-2′-yl)-9,10-anthrachinon,
2,7-Bis-(8′-cyano-2′,6′-dimethyl-oct-2′-yl)-9,10-anthrachinon,
2,6-Bis-(8′-carboxy-2′,6′-dimethyl-oct-2′-yl)-9,10-anthrachinon,
2,7-Bis-(8′-carboxy-2′,6′-dimethyl-oct-2′-yl)-9,10-anthrachinon,
2,6-Bis-(8′-amino-2′,6′-dimethyl-oct-2′-yl)-9,10-anthrachinon,
2,7-Bis-(8′-amino-2′,6′-dimethyl-oct-2′-yl)-9,10-anthrachinon,
2,6-Bis-(8′-carbohydrazido-2′,6′-dimethyl-oct-2′-yl)-9,10-anthrachinon,
2,7-Bis-(8′-carbohydrazido-2′,6′-dimethyl-oct-2′-yl)-9,10-anthrachinon,
2,6 und 2,7-Bis-(4′-carboxy-1′-methyl-cyclo-hex-1′-yl)-9,10-anthrachinon,
2,6 und 2,7-Bis-(4′-methoxycarbonyl-1′-methyl-cyclo-hex-1′-yl)-9,10-anthrachinon,

2,6-Bis-(5′-cyclohexyloxycarbonyl-2′-methyl-pent-2′-yl)-9,10-anthrachinon,
2,7-Bis-(5′-cyclohexyloxycarbonyl-2′-methyl-pent-2′-yl)-9,10-anthrachinon,
2-Methyl-6-(5′-methoxycarbonyl-2′-methyl-pent-2′-yl)-9,10-anthrachinon,
2-Methyl-7-(5′-methoxycarbonyl-2′-methyl-pent-2′-yl)-9,10-anthrachinon,
2-Ethyl-6-(5′-methoxycarbonyl-2′-methyl-pent-2′-yl)-9,10-anthrachinon,
2-Ethyl-7-(5′-methoxycarbonyl-2′-methyl-pent-2′-yl)-9,10-anthrachinon,
2-t-Butyl-6-(5′-carboxy-2′-methyl-pent-2′-yl)-9,10-anthrachinon,
2-t-Butyl-7-(5′-carboxy-2′-methyl-pent-2′-yl)-9,10-anthrachinon,
2-t-Butyl-6-(5′-methoxycarbonyl-2′-methyl-pent-2′-yl)-9,10-anthrachinon,
2-t-Butyl-7-(5′-methoxycarbonyl-2′-methyl-pent-2′-yl)-9,10-anthrachinon,
2-t-Butyl-6-(5′-ethoxycarbonyl-2′-methyl-pent-2′-yl)-9,10-anthrachinon,
2-t-Butyl-7-(5′-ethoxycarbonyl-2′-methyl-pent-2′-yl)-9,10-anthrachinon,
2-t-Butyl-6-(5′-propoxycarbonyl-2′-methyl-pent-2′-yl)-9,10-anthrachinon,
2-t-Butyl-7-(5′-propoxycarbonyl-2′-methyl-pent-2′-yl)-9,10-anthrachinon,
2-t-Butyl-6-(5′-isopropoxycarbonyl-2′-methyl-pent-2′-yl)-9,10-anthrachinon,
2-t-Butyl-7-(5′-isopropoxycarbonyl-2′-methyl-pent-2′-yl)-9,10-anthrachinon,
2-t-Butyl-6-(5′-n-butoxycarbonyl-2′-methyl-pent-2′-yl)-9,10-anthrachinon,
2-t-Butyl-7-(5′-n-butoxycarbonyl-2′-methyl-pent-2′-yl)-9,10-anthrachinon,
2-t-Butyl-6-(5′-n-hexyloxycarbonyl-2′-methyl-pent-2′-yl)-9,10-anthrachinon,
2-t-Butyl-7-(5′-n-hexyloxycarbonyl-2′-methyl-pent-2′-yl)-9,10-anthrachinon,
2-t-Butyl-6-[5′-(2″-ethylhexyloxycarbonyl)-2′-methyl-pent-2′-yl)-9,10-anthrachinon,
2-t-Butyl-7-[5′-(2″-ethylhexyloxycarbonyl)-2′-methyl-pent-2′-yl)-9,10-anthrachinon,
2-t-Butyl-6-(carbohydrazido-2′-ethyl-pent-2′-yl)-9,10-anthrachinon,
2-t-Butyl-7-(carbohydrazido-2′-methyl-pent-2′-yl)-9,10-anthrachinon,
2-t-Butyl-6-(cyclohexyloxycarbonyl-2′-methyl-pent-2′-yl)-9,10-anthrachinon,
2-t-Butyl-7-(cyclohexyloxycarbonyl-2′-methyl-pent-2′-yl)-9,10-anthrachinon,
2-t-Butyl-6-(5′-carbamoyl-2′-methyl-pent-2′-yl)-9,10-anthrachinon,
2-t-Butyl-7-(5′-carbamoyl-2′-methyl-pent-2′-yl)-9,10-anthrachinon,
2-(1′1′,3′,3′-Tetramethylbutyl)-6-(5′-methoxycarbonyl-2′-methyl-pent-2′-yl)-9,10-anthrachinon,
2-(1′,1′,3′,3′-Tetramethylbutyl)-7-(5′-methoxycarbonyl-2′-methyl-pent-2′-yl)-9,10-anthrachinon,
2-Cumyl-6-(5′-methoxycarbonyl-2′-methyl-pent-2′-yl)-9,10-anthrachinon,
2-Cumyl-7-(5′-methoxycarbonyl-2′-methyl-pent-2′-yl)-9,10-anthrachinon,
2-(1′-Methyl-cyclohex-1′-yl)-6-(5′-methoxycarbonyl-2′-methyl-pent-2′-yl)-9,10-anthrachinon,
2-(1′-Methyl-cyclohex-1′-yl)-7-(5′-methoxycarbonyl-2′-methyl-pent-2′-yl)-9,10-anthrachinon,
2-t-Butyl-6-(7′-methoxycarbonyl-2′,6′-dimethyl-hept-2′-yl)-9,10-anthrachinon,
2-t-Butyl-7-(7′-methoxycarbonyl-2′,6′-dimethyl-hept-2′-yl)-9,10-anthrachinon,
2-t-Butyl-6-(7′-cyano-2′,6′-dimethyl-hept-2′-yl)-9,10-anthrachinon,
2-t-Butyl-7-(7′-cyano-2′,6′-dimethyl-hept-2′-yl)-9,10-anthrachinon,
2-t-Butyl-6-(8′-hydroxy-2′,6′-dimethyl-oct-2′-yl)-9,10-anthrachinon,
2-t-Butyl-7-(8′-hydroxy-2′,6′-dimethyl-oct-2′-yl)-9,10-anthrachinon,
2-t-Butyl-6-(8′-acetoxy-2′,6′-dimethyl-oct-2′-yl)-9,10-anthrachinon,
2-t-Butyl-7-(8′-acetoxy-2,′6′-dimethyl-oct-2′-yl)-9,10-anthrachinon,
cis- und trans 2-t-Butyl-6-(4′-methoxycarbonyl-1′-methyl-cyclohex-1′-yl)-9,10-anthrachinon,
cis- und trans 2-t-Butyl-7-(4′-methoxycarbonyl-1′-methyl-cyclohex-1′-yl)-9,10-anthrachinon und
2-5,5-Di-(methoxycarbonyl)-2′-methyl-pent-2′-yl)-9,10-anthrachinon.

Die Verbindungen der Formeln I und Ia können nach an sich bekannten Verfahren hergestellt werden, die dadurch gekennzeichnet sind, dass man

a) ein Anthracen der Formel II

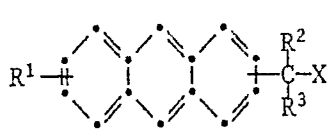

(I)

worin X $-C_nH_{2n+1-k}(Q)_k$ oder $-C_nH_{2n}-Q^1$ ist und $R^1$, $R^2$, $R^3$ Q, $Q^1$, n und k die zuvor angegebene Bedeutung haben, zu einer Verbindung der Formel I oder Ia oxidiert, oder

b) ein substituiertes Benzol der Formel III bzw. IIIa

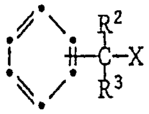 (III)

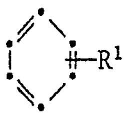 (IIIa)

mit einem Phthalsäureanhydrid der Formeln IV oder IVa

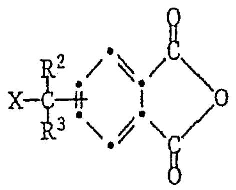 (IV), 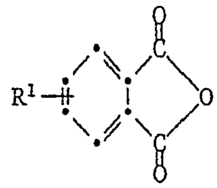 (IVa)

zu Benzophenonverbindungen der Formeln V, Va oder Vb

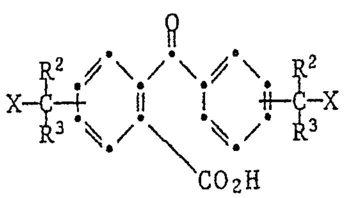 (V), 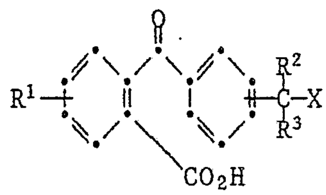 (Va),

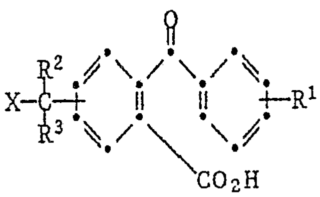 (Vb)

umsetzt, und die Benzophenonverbindungen anschliessend zu den Verbindungen der Formeln I oder Ia cyclisiert.

Die Oxidation der Anthracene kann mittels Chrom- oder Salpetersäure erfolgen, oder katalytisch mit Sauerstoff in Gegenwart von Vanadiumpentoxid. Die Herstellung von Anthracenen der Formel III ist z.B. in der EP-A-0 174 271 beschrieben.

Die Umsetzung der Benzole der Formeln III bzw. IIIa mit den Anhydriden der Formeln IV bzw. IVa ist bekannt und wird um allgemeinen mit $AlCl_3$ als Katalysator durchgeführt. Die Cyclisierung der Benzophenonverbindungen der Formeln V, Va oder Vb kann in bekannter Weise z.B. mit Oleum vergenommen werden. Es kann zweckmässig sein, die funktionelle Gruppe Q bzw. $Q^1$ zu schützen. Es ist auch möglich, Verbindungen der Formeln I so herzustellen, dass man eine funktionelle Gruppe Q oder $Q^1$ in bekannter Weise in andere funktionelle Gruppe umwandelt.

Die substituierten Benzole der Formeln III bzw. IIIa sind bekannt oder können nach üblichen Alkylierungsverfahren für Benzol erhalten werden. Die Phthalsäuren der Formeln IV bzw. IVa sind bekannt oder nach üblichen Verfahren herstellbar. So kann man o-Xylol in bekannter Weise alkylieren bzw. funktionell alkylieren und anschliessend durch Oxidation die entsprechend substituierten Phthalsäuren erhalten, aus denen durch Dehydration die substituierten Anhydride hergestellt werden können. In einen anderen Verfahren kann man z.B. 2-Benzoylaniline unter Friedel-Kafts-Bedingungen mit einem funktionellen Alkylierungsmittel zu einer Verbindung der Formel

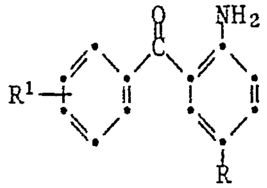

umsetzen. Die Diazotierung und anschliessende Umsetzung mit NaCN/CuCN führt zum Nitril, dessen Hydrolyse die Carbonsäure ergibt, die zu Verbindungen der Formel I bzw. Ia cyclisiert werden kann. Geeignete funktionelle Alkylierungsmittel sind z.B. tertiäre Alkohole und verzweigte Alkene mit funktionellen Gruppen Q bzw. $Q^1$. Die Verbindungen der Formeln V, Va und Vb sind neu und bilden einen weiteren Gegenstand der Erfindung.

Die Verbindungen der Formel I eignen sich zur Herstellung von lichtempfindlichen gehärteten Epoxidharzen. Ein weiterer Gegenstand der Erfindung ist eine härtbare Zusammensetzung, enthaltend

a) mindestens ein Epoxidharz mit durchschnittlich mehr als einer Epoxidgruppe im Molekül,

b) gegebenenfalls einen Härter für das Epoxidharz,

c) ein Anthrachinon der Formel I gemäss Anspruch 1 oder Mischungen hiervon und

d) mindestens ein primäres oder sekundäres aliphatisches Amin, das im aliphatischen Rest mindestens eine Hydroxylgruppe enthält.

Das Epoxidharz enthält bevorzugt durchschnittlich mindestens 2 Epoxidgruppen im Molekül.

Als Epoxidharze kommen vor allem solche mit durchschnittlich mehr als einer an ein Heteroatom (z.B. Schwefel, vorzugsweise Sauerstoff oder Stickstoff) gebundenen Glycidylgruppe, β-Methylglycidylgruppe oder 2,3-Epoxycyclopentylgruppe in Frage; genannt seien insbesondere Bis-(2,3-epoxycyclopentyl)-ether; Di- bzw. Polyglycidylether von mehrwertigen aliphatischen Alkoholen, wie 1,4-Butandiol, oder Polyalkylenglykolen, wie Polypropylenglykole; Di- oder Polyglycidylether von cycloaliphatischen Polyolen, wie 2,2-Bis-(4-hydroxycyclohexyl)-propan; Di- bzw. Polyglycidylether von mehrwertigen Phenolen, wie Resorcin, Tris-(p-hydroxyphenyl)-methan, 2,2-Bis-(p-hydroxyphenyl)-propan (= Diomethan), 2,2-Bis-(4′-hydroxy-3′,5′-dibromphenyl)-propan, 1,1,2,2-Tetrakis (p-hydroxyphenyl)-ethan, oder von unter sauren Bedingungen erhaltenen Kondensationsprodukten von Phenolen mit Formaldehyd wie Phenol-Novolake und Kresol-Novolake; Di- bzw. Poly-(β-methylglycidyl)-ether der oben angeführten mehrwertigen Alkohole oder mehrwertigen Phenole; Polyglycidylester von mehrwertigen Carbonsäuren, wie Phthalsäure, Terephthalsäure, $\Delta^4$-Tetrahydrophthalsäure und Hexahydrophthalsäure; N-Glycidyl-derivate von Aminen, Amiden und heterocyclischen Stickstoffbasen, wie N,N-Diglycidylanilin, N,N-Diglycidyltoluidin, N,N,O-Triglycidyl-p-aminophenol, N,N,N′,N′-Tetraglycidyl-bis-(p-aminophenyl)-methan; Triglycidyl-isocyanurat; N,N′-Diglycidylethylenharnstoff; N,N′-Diglycidyl-5,5-dimethylhydantoin, N,N′-Diglycidyl-5-isopropylhydantoin, N,N-Methylen-bis-(N′,N′-diglycidyl)-5,5-dimethylhydantoin, 1,3-Bis-(N-glycidyl-5,5-dimethylhydantoin)-2-glycidyloxypropan; N,N′-Diglycidyl-5,5-dimethyl-6-isopropyl-5,6-dihydro-uracil.

Eine bevorzugte Gruppe von Epoxidharzen sind glycidylierte Novolake, Hydantoine, Aminophenole, Bisphenole oder aromatische Diamine. Besonders bevorzugte Zusammensetzungen enthalten als Epoxidharz einen glycidylierten Kresolnovolak, Bisphenol-A-diglycidylether, mit Bisphenol-A vorverlängerten Bisphenol-A-diglycidylether, Hydantoin-N,N′-bisglycid, Propylen-1,3-bis-hydantoin-2-hydroxytriglycid, p-Aminophenoltriglycid, Diaminodiphenylmethan-tetraglycid oder Mischungen hiervon.

Geeignet sind auch vorreagierte Addukte solcher Epoxide mit Härtern für Epoxide, z.B. das erwähnte Addukt aus Bisphenol-A-diglycidylether und Bisphenol-A.

Als Härter für Epoxidharze kommen saure oder basische Verbindungen in Frage. Als geeignete Härter seien z.B. genannt: Amine wie aliphatische, cycloaliphatische oder aromatische, primäre, sekundäre und tertiäre Amine, z.B. Ethylendiamin, Hexamethylendiamin, Trimethylhexamethylendiamin, Diethylentriamin, Triethylentetramin, Tetraethylenpentamin, N,N-Dimethylpropylen-1,3-diamin, N,N-Diethylpropylendiamin-1,3, 2,2-Bis-(4′-aminocyclohexyl)-propan, 3,5,5-Trimethyl-3-(aminomethyl)-cyclohexylamin ("Isophorondiamin"), Mannichbasen, wie 2,4,6-Tris-(dimethylaminomethyl)-phenol; m-Phenylendiamin, p-Phenylendiamin, Bis-(4-aminophenyl)-methan, Bis-(4-aminophenyl)-sulfon, m-Xylylendiamin; Addukte von Acrylnitril oder Monoepoxiden, wie z.B. Ethylenoxid oder Propylenoxid an Polyalkylenpolyamine, wie z.B. Diethylentriamin oder Triethylentetramin; Addukte aus Polyaminen, wie z.B. Diethylentriamin oder Triethylentetramin, im Ueberschuss und Polyepoxiden, wie z.B. Diomethanpolyglycidylethern; Addukte aus Monophenolen oder Polyphenolen und Polyamiden; Polyamide, insbesondere solche aus aliphatischen Polyaminen, wie z.B. Diethylentriamin oder Triethylentetramin, und di- oder trimerisierten ungesättigten Fettsäuren, wie z.B. dimerisierte Leinölfettsäure (VERSAMID®); Polysulfide (THIOKOL®); Anilin-Formaldehyde; mehrwertige Phenole, z.B. Resorcin, 2,2-Bis-(4-hydroxyphenyl)-propan oder Phenol-Formaldehyd-Harze; mehrbasische Carbonsäuren und ihre Anhydride, z.B. Phthalsäureanhydrid, $\Delta^4$-Tetrahydrophthalsäureanhydrid, Hexahydrophthalsäureanhydrid, 4-Methylhexahydrophthalsäureanhydrid, 3,6-Endomethylen-$\Delta^4$-tetrahydrophthalsäureanhydrid, 4-Methyl-3,6-endomethylen-$\Delta^4$-tetrahydrophthalsäureanhydrid (= Methylnadicanhydrid), 3,4,5,6,7-Hexachlor-3,6-endomethylen-$\Delta^4$-tetrahydrophthalsäureanhydrid, Bernsteinsäureanhydrid, Adipinsäureanhydrid, Trimethyladipinsäure-

anhydrid, Azelainsäureanhydrid, Sebacinsäureanhydrid, Maleinsäureanhydrid, Dodecylbernsteinsäureanhydrid; Pyromellitsäuredianhydrid, Trimellitsäureanhydrid, Benzophenontetracarbonsäuredianhydrid oder Gemische solcher Anhydride.

Eine bevorzugte Gruppe von Härtern sind Novolake, Polyamine und Carbonsäureanhydride.

Die erfindungsgemässe Zusammensetzung kann auch Härtungsbeschleuniger oder thermische bzw. photochemische Härtungskatalysatoren enthalten. Beispiele sind: tertiäre Amine, deren Salze oder quaternäre Ammoniumverbindungen, z.B. 2,4,6-Tris-(dimethylaminomethyl)phenol, Benzyldimethylamin, 2-Ethyl-4-methylimidazol, Triamylammoniumphenolat; Mono- oder Polyphenole wie Phenol oder Diomethan oder Salicylsäure; Dicyandiamid; Bortrifluorid und sein Komplexe mit organischen Verbindungen, wie $BF_3$-Ether-Komplexe und $BF_3$-Amin-Komplexe, z.B. $BF_3$-Monoethylamin-Komplex; Acetoacetanilid-$BF_3$-Komplex; Phosphorsäure; Triphenylphosphit. Als photoempfindliche Härtungsbeschleuniger eignen sich Oniumsalze oder Metallkomplexe wie z.B. Diazoniumsalze aromatischer Amine, Triphenylsulfonium- oder Diphenyliodoniumsalze oder Cyclopentadienyleisenarensalze.

Härtungsbeschleuniger und -katalysatoren werden üblicherweise in einer Menge von 0,1-10 Gew.-% zugegeben, bezogen auf das Epoxidharz. Härter für Epoxidharze werden im allgemeinen in äquimolaren Mengen verwendet, bezogen auf die Epoxidgruppen und funktionellen Gruppen eines Härters.

Die Zusammensetzung enthält das Anthrachinon der Formel I bevorzugt in einer Menge von 0,1-1, besonders 0,2-0,8 Mol/kg Epoxidharz, und das Hydroxylgruppen enthaltende Amin bevorzugt in einer Menge von 0,1-1,2, besonders 0,3-1 Mol/kg Epoxidharz. Zusätzliche Härter sind bevorzugt in einer Menge von 0,1-0,5, besonders 0,1-0,3 Mol/kg Epoxidharz enthalten.

Bei den Aminen der Komponente d) kann es sich um Hydroxylgruppen enthaltende aliphatische Amine mit 2 bis 30, vorzugsweise 2 bis 20 C-Atomen und mit 1 bis 3, vorzugsweise 1 Hydroxylgruppen im ali phatischen Rest handeln. Der aliphatische Rest kann linear oder verzweigt sein und durch -O- oder Aminogruppen unterbrochen sein. Bevorzugt enthält der aliphatische Rest primäre OH-Gruppen. In einer bevorzugten Ausführungsform entspricht das Amin der Formel VI

$$H-\underset{}{\overset{R^7}{\underset{|}{N}}}-C_yH_{2y+1-x}(OH)_x \qquad (VI),$$

worin $R^7$ H, lineares oder verzweigtes $C_1$-$C_{18}$-Alkyl, $C_3$-$C_7$-Cycloalkyl, $C_6$-$C_{10}$-Aryl, $C_7$-$C_{18}$-Alkaryl, $C_7$-$C_{12}$-Aralkyl, $C_8$-$C_{18}$-Alkaralkyl oder die Gruppe $-C_yH_{2y+1-x}OH_x$ bedeutet, x für eine Zahl von 1 bis 3 und y für eine Zahl von 2 bis 12 stehen, wobei die Gruppe $C_yH_{2y}$ durch ein oder mehrere -O- oder $-NR^7-$ unterbrochen sein kann.

$R^7$ enthält als Alkyl bevorzugt 1 bis 12 und besonders 1 bis 6 C-Atome. $R^7$ ist als Alkyl bevorzugt Methyl oder Ethyl. Insbesondere stellt $R^7$ H dar. Als Cycloalkyl enthält $R^7$ vorzugsweise 5 oder 6 Ring-C-Atome und ist z.B. Cyclopentyl oder Cyclohexyl.

$R^7$ kann in der Bedeutung von Aryl z.B. Naphthyl und besonders Phenyl sein. $R^7$ in der Bedeutung von Aralkyl ist besonders $C_7$-$C_{18}$-Alkylphenyl, zum Beispiel Methyl-, Ethyl-, Dimethyl-, n- und i-Propyl-, n-, i- und t-Butyl-, Pentyl-, Hexyl-, Octyl-, Nonyl-, Decyl- und Dodecylphenyl. Bei $R^7$ in der Bedeutung von Aralkyl kann es sich um 1- oder 2-Phenyleth-1-yl oder besonders Benzyl handeln. Wenn $R^7$ Alkaralkyl ist, handelt es sich bevorzugt um Alkylbenzyl mit insbesondere 8 bis 14 C-Atomen, z.B. Methyl-, Ethyl-, Dimethyl-, n- und i-Propyl-, n-, i- und t-Butyl-, Pentyl- und Hexylbenzyl. Sofern die Gruppe $C_yH_{2y}$ durch -O- unterbrochen ist, kann es sich um Oxaalkylenreste handeln, die z.B. der Formel $-R^8-(OR^9)_t-$ entsprechen können, worin $R^8$ und $R^9$ unabhängig voneinander lineares oder verzweigtes $C_2$-$C_6$-Alkylen und t eine Zahl von 2 bis 6 sind. Sofern die Gruppe $C_yH_{2y}$ durch $-NR^7-$ unterbrochen ist, kann die Gruppe vorzugsweise der Formel $-(-R^{10}-NH-)_s-R^{11}-$ entsprechen, worin $R^{10}$ lineares oder verzweigtes $C_2$-$C_6$-Alkylen, bevorzugt Ethylen, $R^{11}$ lineares oder verzweigtes $C_1$-$C_{10}$-, vorzugsweise $C_2$-$C_6$-Alkylen und s eine Zahl von 1 bis 3 sind. In Formel VI steht y besonders für eine Zahl von 2 bis 7.

Eine bevorzugte Untergruppe sind solche Amine der Formel VI, worin $R^7$ für H steht, y eine Zahl von 2 bis 7 und x eine Zahl von 1 bis 3 bedeuten.

Beispiele für Amine der Formel VI sind: Ethanolamin, 1-Amino-2-hydroxypropan, 1-Amino-3-hydroxypropan, 1-Amino-4-hydroxybutan, 1-Amino-5-hydroxypentan, 1-Amino-6-hydroxyhexan, Aminotrimethylolmethan, Aminodimethylolmethan, Aminomethyldimethylolmethan, Aminomethyltrimethylolmethan, Hydroxyethoxyethylamin, Hydroxypropoxyethylamin, N-(Hydroxyethyl)ethylendiamin, N-(Hydroxyethyl)-diethylentriamin, $H_2N(CH_2CH_2O-)_{\overline{2-6}}H$.

Primäre aliphatische Hydroxylgruppen enthaltende Amine sind Härter für Epoxidharze, wobei lineare Polymere erhalten werden, wenn Epoxidharze mit 2 Epoxidgruppen verwendet werden. Bei Mitverwendung anderer Härter werden vernetzte Polymere erhalten.

In den erfindungsgemässen Zusammensetzungen werden vorzugsweise Anthrachinone der Formel I verwendet, wenn $R^1$ für H steht, $R^2$ und $R^3$ unabhängig voneinander Methyl oder Ethyl darstellen k = 1 und n eine Zahl von 1 bis 5 sind, und Q $-CO-NR^4-NH_2$ bedeutet, worin $R^4$ für H oder Methyl steht.

Ganz besonders entsprechen in den erfindungsgemässen Zusammensetzungen die Anthrachinone der

Formel

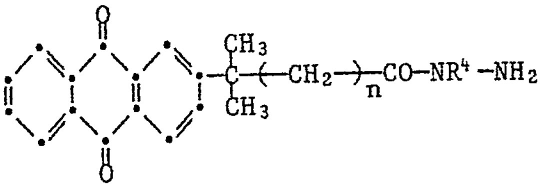

worin n eine Zahl von 1 bis 3 und $R^4$ H oder Methyl sind.

Bei Verwendung der monofunktionellen Anthrachinone der Formel I werden vorteilhaft Epoxidharze mit mindestens 3 Epoxidgruppen im Molekül verwendet, z.B. epoxidierte Novolake, um vernetzte Epoxidharze zu erhalten.

Die erfindungsgemässen Zusammensetzungen sind härtbar, wobei die gehärteten bzw. vernetzten Epoxidharze lichtempfindlich sind. Auf den belichteten Stellen der Oberfläche können durch stromlose Metallabscheidung dünne Schichten von Metallen wie z.B. Kupfer abgeschieden werden.

Die Härtung der Zusammensetzung erfolgt in bekannter Weise, wobei vor oder mit der Härtung eine Formgebung nach den üblichen Formgebungsverfahren möglich ist, z.B. die Herstellung von Beschichtungen auf einem Trägermaterial durch Spritzen, Streichen oder Rakeln, oder die Herstellung von Formteilen mittels Giesstechniken, oder die Herstellung von Verbundkörpern mittels Tränk- und Press-verfahren.

Das Hydroxylgruppen enthaltende Amin der Komponente d) kann in einer bevorzugten Ausführungsform mit dem Epoxidharz der Komponente a) zu Addukten vorreagiert und dann mit einem als Härter wirkenden Anthrachinon der Formel I gegebenenfalls zusammen mit Härtern für Epoxidharze vermischt und vernetzt werden. Geeignete Härter von Anthrachinonen der Formel I sind insbesondere solche difunktionelle Anthrachinone, worin in Formel I $R^1$ einen Rest $-CR^2R^3C_nH_{2n}(Q)$ bedeutet und Q für -OH, $-NHR^4$ oder -COOH steht, oder worin $R^1$, H, Alkyl, Aralkyl oder Cycloalkyl bedeutet und Q $-CO-NR^4-NH_2$, $-OCOR^5$, $-NHR^4$ oder

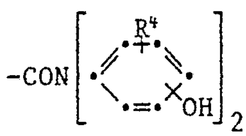

ist. Besonders bevorzugt sind die Hydrazide der Monocarbonsäuren der Formel I.

Bei Verwendung monofunktioneller Anthrachinone der Formel I, worin $R^1$ H, Alkyl, Aralkyl oder Cycloalkyl und Q -OH, $-NHR^4$ oder -COOH sind, kann es vorteilhaft sein, einen Härter für Epoxidharze mitzuverwenden, besonders einen Novolak-, Amin- oder Anhydridhärter. Zweckmässig wird das monofunktionelle Anthrachinon zusammen mit dem -OH enthaltenden Amin und dem Epoxid vorreagiert und darauf mit einem Härter vermischt und ausgehärtet.

Neben einer stufenweisen Härtung ist auch das Vermischen aller Komponenten und die darauffolgende Härtung möglich.

Das Vermischen der Komponenten erfolgt nach üblichen Verarbeitungsmethoden gegebenenfalls zusammen mit einem Lösungsmittel. Es können weitere für die Verarbeitung oder die Verbesserung der Eigenschaften der gehärteten Epoxidharze übliche Zusätze zugegeben werden, z.B. Weichmacher, Farbstoffe, Pigmente, Füllstoffe, Formtrennungsmittel oder H-Donoren. Für die Abscheidung von Metallen kann ein Gehalt an Metallsalzen oder Metallkomplexen der Gruppen Ib oder VIII des periodischen Systems der Elemente vorteilhaft sein, z.B. in einer Menge von 0,01 bis 10 Gew.-%, bezogen auf die Zusammensetzung. Die Härtung wird im allgemeinen bei Temperaturen von 20 bis 200° C, besonders 50 bis 150° C vorgenommen. Die gehärteten Zusammensetzungen sind ein weiterer Gegenstand der Erfindung.

Die gehärteten Zusammensetzungen sind lichtempfindlich. Die belichteten Teile erscheinen dunkler als die unbelichteten Teile. Auf den belichteten Teilen können mit konventionellen Metallabscheidungsbädern (siehe z.B. US-PS 4 510 279), besonders solchen mit z.B. Nickel- oder Kupfersalzen, direkt Metalle abgeschieden werden. So können z.B. gedruckte Schaltungen hergestellt werden. Die belichteten Epoxidharze können auch für optische Speicherungen verwendet werden.

Ein weiterer Gegenstand der Erfindung ist die Verwendung einer gehärteten erfindungsgemässen Zusammensetzung zur Herstellung von metallischen Ueberzügen oder Abbildungen durch stromlose Metallabscheidung nach einer vollständigen oder teilweisen Belichtung der Oberfläche.

Die Belichtung wird bevorzugt mit UV-Licht vorgenommen. Es können beliebige Lichtquellen eingesetzt werden, wobei die Verwendung von UV-Lampen bevorzugt ist. Geeignete Lichtquellen sind z.B. Xenonlampen, Metallhalogenidlampen und besonders Quecksilberhoch- und -mitteldrucklampen.

Zur Herstellung der metallischen Ueberzüge oder Abbildungen kann man so vorgehen, dass man die erfindungsgemäss, sich gegebenenfalls als Schicht auf einem Trägermaterial befindlich erfindungsgemässe Zusammensetzung härtet, darauf flächenmässig oder durch eine Bildvorlage belichtet und danach mit einem Metallabscheidungsbad behandelt.

Die Mitverwendung eines Metallsalzes oder Metallkomplexes ist überflüssig. Die abgeschiedenen Metalle haften fest auf der Epoxidharzoberfläche; eine Vorbehandlung ist nicht notwendig. Ferner weisen die gehärteten Zusammensetzung erhöhte Glasumwandlungstemperaturen auf.

Die nachfolgenden Beispiele erläutern die Erfindung näher. Teile sind Gewichtsteile. Ether ist Diethylether.

A) Herstellung von substituierten Anthrachinonen

Beispiel 1: 5-(2′-Anthrachinoyl)-5-methylhexansäuremethylester

a) 8,9 Teile Anthracen, 14,2 Teile 5-Methyl-5-hexancarbonsäuremethylester und 26,1 Teile Zinntetrachlorid werden mit 50 Teilen Xylol vermischt und 24 Stunden im Wasserdampfbad erhitzt. Die Reaktionsmischung wird in 250 Teile 2N Salzsäure gegossen und die organische Phase mit Ether extrahiert. Die Etherlösung wird nach einander mit Wasser, wässrigem Natriumcarbonat und Wasser gewaschen und der Ether abgedampft. Der Rückstand wird in Methanol aufgenommen und das ungelöste Anthracen (3,7 Teile) abfiltriert. Von der Methanollösung wird unter vermindertem Druck das Methanol abdestilliert. Der Rückstand wird destilliert. Man erhält eine Fraktion mit einem Siedepunkt von 220° C/0,7 mbar. Die Kristallisation der Fraktion aus Petrolether (40-60° C) ergibt 5-(2′-Anthracenyl)-5-methylhexansäuremethylester mit einem Schmelzpunkt von 82-84° C.

b) 7,1 Teile dieser Verbindung und 50 Teile Eisessig werden zusammengegeben, gerührt und auf dem Wasserdampfbad erhitzt. Während 30 Minuten wird eine Lösung von 5 Teilen Chromtrioxid in 5 Teilen Wasser und 20 Teilen Eisessig zugetropft. Danach wird noch 30 Minuten erhitzt. Darauf wird der Eisessig am Rotationsverdampfer unter vermindertem Druck abdestilliert. Der Rückstand wird mit Wasser verdünnt und die organische Phase mit Ether extrahiert, die Etherlösung mit Wasser gewaschen und der Ether abgedampft. Die Destillation des Rückstandes ergibt eine bei 230-240° C/0,4 mbar siedende Fraktion. Das Destillat wird mit Petrolether (40-60° C), der wenig Ether enthält, verdünnt und der Niederschlag abfiltriert und getrocknet. Man erhält 5-(2′-Anthrachinoyl)-5-methylhexancarbonsäuremethylester mit einem Schmelzpunkt von 72-74° C.

c) Die alkalische Hydrolyse ergibt 5-(2′-Anthrachinoyl)-5-methylhexancarbonsäure mit einem Schmelzpunkt von 125-127° C (aus Ether umkristallisiert).

Beispiel 2: 5-(2′-Anthrachinoyl)-5-methylpentan-1-nitril

a) 5-(2′-Anthracenyl)-5-methylhexancarbonsäuremethylester gemäss Beispiel 1a ergibt nach der alkalischen Hydrolyse 5-(2′-Anthracenyl)-5-methylhexancarbonsäure mit einem Schmelzpunkt von 149-151° C (aus Eisessig/Wasser umkristallisiert).

b) 8,2 Teile dieser Verbindung werden mit 8,2 Teilen Harnstoff vermischt und 3,5 Stunden bei 190° C gehalten. Die abgekühlte Mischung wird mit Aceton aufgenommen und filtriert. Das Aceton wird abgedampft und der Rückstand mit Essigsäureethylester aufgenommen. Die Lösung wird mit wässrigem Natriumbicarbonat und Wasser gewaschen. Danach wird der Essigsäureethylester abgedampft und der Rückstand mit Petrolether (40-60° C) aufgenommen. Der Niederschlag wird abfiltriert und getrocknet. Man erhält 5-(2′-Anthracenyl)-5-methylhexancarbonsäureamid vom Schmelzpunkt 142-144° C.

c) 1,7 Teile dieser Verbindung werden zusammen mit 1,6 Teilen Phosphorpentoxid 4 Stunden bei 195° C erhitzt. Nach dem Abkühlen wird die Reaktionsmasse mit Ethylacetat filtriert und das Ethylacetat verdampft. Der Rückstand wird bei 220° C/0,13 mbar einer Kurzwegdestillation unterworfen und das Destillat aus Ethylacetat umkristallisiert. Man erhält 5-(2′-Anthracenyl)-5-methylpentan-1-nitril mit einem Schmelzpunkt von 111-115° C.

d) 0,2 Teile dieser Verbindung werden in 5 Teilen Aceton gelöst und dann mit 2 Teilen Jones Reagenz behandelt. Nach 5 Minuten Stehen wird die Reaktionsmischung in Wasser gegossen. Man extrahiert mit Ether, wäscht die Etherlösung mit Wasser, verdampft den Ether und unterwirft den Rückstand einer Kurzwegdestillation. Man erhält 5-(2′-Anthrachinoyl)-5-methylpentan-1-nitril als rotes Oel.

Elementaranalyse (Gew.-%):

| | C | H | N |
|---|---|---|---|
| Gef.: | 79,44 | 6,23 | 4,06 |
| Ber.: | 79,47 | 6,03 | 4,41 |

Beispiel 3: N-[5-(2′-Anthrachinoyl)-5-methylhex-1-yl]-acetamid

a) 90 Teile Borantetrahydrofuran-Komplex (1M Lösung in Tetrahydrofuran) werden gerührt und 5 Teile Verbindung gemäss 2b portionsweise während 30 Minuten zugegeben. Danach wird noch 3 Stunden am Rückfluss erhitzt, abgekühlt und 60 Teile 6M Salzsäure zugegeben. Das Tetrahydrofuran wird unter vermindertem Druck entfernt, die wässrige Phase mit Sodalösung behandelt und danach mit Ethylacetat extrahiert. Die Ethylenacetatlösung wird mit Wasser gewaschen, das Ethylacetat verdampft und der Rückstand, in 10 Teilen Toluol gelöst, mit 2 Teilen Essigsäureanhydrid behandelt. Danach wird noch 30 Minuten auf dem Dampfbad erwärmt und die flüchtigen Anteile am Rotationsverdampfer unter vermindertem Druck entfernt. Der ölige Rückstand wird mit einer Ether/Petrolether (40-60° C) digeriert. Der kristalline Niederschlag wird abfiltriert und getrocknet. Man erhält N-[5-(2′-Anthracenyl)-5-methylhex-1-yl]acetamid vom Schmelzpunkt 90-93° C.

b) 0,5 Teile dieser Verbindung werden in 25 Teilen Aceton gelöst und mit Jones-Reagenz behandelt, bis eine orange-braune Farbe bestehen bleibt. Das Aceton wird unter vermindertem Druck entfernt und der Rückstand mit Wasser versetzt. Man extrahiert mit Ether, wäscht mit Wasser und verdampft den Ether. Man erhält ein rotes Oel, das mit Ether und wenig Aceton verdünnt wird. Der gebildete Niederschlag wird abfiltriert und getrocknet. Man erhält n-[5-(2′-Anthrachinoyl)-5-methylhex-1-yl]acetamid vom Schmelzpunkt 112-113° C.

Beispiel 4: 8-(2′-Anthrachinoyl)-2,6-dimethyloctan-1-ol

a) 8,9 Teile Anthracen, 19,8 Teile Citronellolessigsäureester, 13 Teile Zinntetrachlorid und 1,5 Teile Trifluormethansulfonsäure werden mit 50 Teilen p-Xylol vermischt und 8 Stunden auf einem Dampfbad erhitzt. Die abgekühlte Reaktionsmischung wird in verdünnte Salzsäure gegossen und darauf mit Ether extrahiert. Der Extrakt wird mit Wasser gewaschen, eingedampft und destilliert. Man erhält eine bei 220-280° C/0,78 mbar siedende Fraktion, die an Silica mit Petrolether (40-60° C) chromatographiert wird. Die Elution mit Petrolether, der 2 Vol.-%, Ether enthält, ergibt 8-(2′-Anthracenyl)-2,6-dimethyl-1-acetoxy-octan als fahlgelbes Oel.

Elementaranalyse (Gew.-%):

| | C | H |
|---|---|---|
| Gef.: | 82,65 | 8,76 |
| Ber.: | 82,94 | 8,57 |

b) Die Verbindung wird gemäs Beispiel 2d mit Jones-Reagenz oxidiert und aufgearbeitet. Man erhält 8-(2′-Anthrachinoyl)-2,6-dimethyl-1-acetoxy-octan als viskoses gelbes Oel.

Elementaranalyse (Gew.-%):

| | C | H |
|---|---|---|
| Gef.: | 76,80 | 7,17 |
| Ber.: | 76,82 | 7,44 |

c) Die alkalische Hydrolyse dieser Verbindung ergibt 8-(2'-Anthrachinoyl)-2,6-dimethyl-octan-1-ol als gelbes viskoses Oel.

Elementaranalyse (Gew.-%):

| | C | H |
|---|---|---|
| Gef.: | 79,01 | 7,77 |
| Ber.: | 79,09 | 7,74 |

Beispiel 5: 2,6-Bis(5-carboxy-2-methylpent-2-yl)anthrachinon

a) 26,1 Teile wasserfreies Zinntetrachlorid werden in 100 Teilen p-Xylol gelöst. Zu der Lösung werden 1,5 Teile Trifluormethansulfonsäure, 14,2 Teile 5-Methylhex-5-encarbonsäuremethylester und 17,8 Teile Anthracen gegeben. Die Mischung wird 8 Stunden auf dem Wasserdampfbad erhitzt und nach dem Abkühlen in 500 Teile Wasser gegossen. Man extrahiert mit Ether, wäscht die Etherlösung mit Wasser und verdampft den Ether. Der Rückstand wird mit 300 Teilen Methanol versetzt, zum Rückfluss erhitzt und dann filtriert. Das Filtrat wird mit 1 Teil p-Toluolsulfonsäure versetzt und 4 Stunden am Rückfluss erhitzt und darauf das Methanol abdestilliert. Der Rückstand wird mit Ether aufgenommen, mit wässrigem Natriumbicarbonat und Wasser gewaschen und der Ether abdestilliert. Die Destillation ergibt eine erste bei 210-240° C/0,5 mbar siedende Fraktion, die die Verbindung gemäss Beisiel 1a enthält. Eine zweite bei 240-290° C/0,5 mbar siedende Fraktion enthält eine Mischung von 2,6- und 2,7-Bis(5-carboxy-2-methyl-pent-2-yl)anthracen. Die fraktionierte Kristallisation aus Methanol ergibt die reinen kristallinen 2,6- und 2,7-Isomeren mit Schmelzpunkten von 111-113° C bzw. 94-96° C.

b) 5 Teile des 2,6-Isomeren in 25 Teilen Eisessig gelöst werden auf einem Wasserdampfbad erhitzt. Zu der Lösung werden 3 Teile Chromtrioxid, gelöst in einer Mischung aus 3 Teilen Wasser und 10 Teilen Eisessig, während 30 Minuten zugegeben. Man erwärmt noch 30 Minuten auf dem Wasserdampfbad und verdünnt mit Wasser. Man extrahiert mit Ether, wäscht die Etherlösung mit Wasser und dampft den Ether ab. Der ölige Rückstand wird mit Methanol digeriert, der kristalline Niederschlag abfiltriert und getrocknet. Man erhält 2,6-Bis(5-methoxycarbonyl-2-methyl-pent-2-yl)anthrachinon vom Schmelzpunkt 102-104° C.

c) Die alkalische Hydrolyse ergibt 2,6-Bis(5-carboxy-2-methylpent-2-yl)anthrachinon vom Schmelzpunkt 222-224° C (Umkristallisation aus Essigsäure).

Beispiel 6: 2,7-Bis(5-carboxy-2-methylpent-2-yl)anthrachinon

a) Das gemäss Beispiel 5a erhaltene 2,7-Isomere wird wie in Beispiel 5b oxidiert und isoliert. Man erhält 2,7-Bis(5-methoxycarbonyl-2-methylpent-2-yl)anthrachinon als gelbes viskoses Oel.

Elementaranalyse (Gew.-%):

| | C | H |
|---|---|---|
| Gef.: | 73,51 | 7,22 |
| Ber.: | 73,15 | 7,37 |

b) Die alkalische Hydrolyse ergibt 2,7-Bis(5-carboxy-2-methylpent-2-yl)anthrachinon vom Schmelzpunkt 145-147° C (Umkristallisation aus Ether).

Beispiel 7: 3-(2'-Anthrachinoyl)-3-methylbutancarbonsäuremethylester

a) Zu einer gerührten Suspension von 13,3 Teilen Aluminiumtrichlorid in 78,1 Teilen Benzol werden tropfenweise während einer Stunde 11,4 Teile 2-Methylcrotonsäuremethylester gegeben, wobei die Temperatur zwischen 25 und 30° C gehalten wird. Die gebildete homogene Lösung wird noch 6 Stunden bei dieser Temperatur gerührt und dann in 400 Teile Wasser gegossen. Das gebildete Oel wird mit Ether extrahiert, der Extrakt mit Wasser gewaschen, der Ether verdampft und der Rückstand destilliert. Man

erhält 3-Phenyl-3-methylbutancarbonsäuremethylester.

b) Zu 19,2 Teilen dieser Verbindung und 5 Teilen Schwefelkohlenstoff werden 14,8 Teile Phthalsäureanhydrid und 40,2 Teile Aluminiumchlorid gegeben. Das Gemisch wird 20 Stunden unter Rückfluss erwärmt. Man fügt ausreichend Wasser zur Zerstörung des $AlCl_3$-Komplexes zu, und extrahiert mit Ether. Der Extrakt wird mit Wasser gewaschen, der Ether abdestilliert und der Rückstand mit einer Mischung Toluol und Petrolether aufgenommen, der kristalline Niederschlag abfiltriert und getrocknet. Man erhält 2-Carboxy-4′-(3-methoxycarbonyl-2-methylprop-2-yl)-benzophenon mit einem Schmelzpunkt von 118-120°C.

c) 3,4 Teile dieser Verbindung werden in 50 Teilen Schwefelkohlenstoff und 1,2 Teilen Thionylchlorid suspendiert und 2 Stunden zum Rückfluss erhitzt. Nachdem sich die Säure gelöst hat, werden 1,3 Teile Aluminiumtrichlorid zugegeben und weitere 20 Stunden am Rückfluss erhitzt. Man gibt Wasser zu, extrahiert mit Ether, wäscht den Extrakt mit Wasser und wässrigem Natriumbicarbonat und wieder mit Wasser. Der Ether wird abdestilliert und der Rückstand mit einer Mischung aus Ether und Petrolether (40-60°C) aufgenommen. Der kristalline Niederschlag wird abfiltriert und getrocknet. Man erhält 3-(2′-Anthrachinoyl)-3-methylbutancarbonsäuremethylester mit einem Schmelzpunkt von 103-105°C.

Beispiel 8: 5-(2′-Anthrachinoyl)-5-methylhexancarbonsäuremethylester

a) 300 Teile 98%-ige Schwefelsäure, 64 Teile Methanol, 71 Teile 5-Methylhex-5-encarbonsäuremethylester und 53 Teile o-Xylol werden 3 Tage bei Raumtemperatur gerührt und dann in 2000 Teile Wasser gegossen. Man extrahiert mit Ether, wäscht den Extrakt mit Wasser und verdampft den Ether. Der Rückstand wird in 500 Teilen Methanol gelöst, mit einem Teil p-Toluolsulfonsäure versetzt und 6 Stunden am Rückfluss erhitzt. Danach wird das Methanol abdestilliert, der Rückstand in Ether aufgenommen und erst mit wässrigem Natriumbicarbonat und dann mit Wasser gewaschen. Der Ether wird abgedampft und der Rückstand destilliert. Man erhält 5-(3,4-Dimethylphenyl)-5-methylhexancarbonsäuremethylester mit einem Siedepunkt von 176-182°C/16 mbar.

Elementaranalyse (Gew.-%):

| | C | H |
|---|---|---|
| Gef.: | 77,65 | 9,57 |
| Ber.: | 77,38 | 9,74 |

b) Die alkalische Hydrolyse dieses Esters ergibt 5-(3,4-Dimethylphenyl)-5-methylhexancarbonsäure mit einem Schmelzpunkt von 71-73°C (Umkristallisation aus Petrolether).

c) Zu einer gerührten und am Rückfluss gehaltenen Lösung dieser Verbindung in 600 Teilen Wasser und 10,1 Teilen KOH werden portionsweise während 3 Stunden 158 Teile $KMnO_4$ gegeben. Es wird 1 Stunde weiter am Rückfluss erhitzt, abgekühlt und das gebildete $MnO_2$ abfiltriert. Das Filtrat wird mit Salzsäure angesäuert, unter Vakuum am Rotationsverdampfer zur Trockene eingedampft und der Rückstand mit Aceton extrahiert. Der Extrakt wird filtriert, das Aceton verdampft und der Rückstand mit einer Mischung aus Ethylacetat und Petrolether (40-60°C) aufgenommen. Der kristalline Niederschlag wird abfiltriert und getrocknet. Man erhält 4-(5-Carboxy-2-methylpent-2-yl)phthalsäure mit einem Schmelzpunkt von 154-158°C.

d) 23,5 Teile dieser Verbindung werden 3 Stunden bei 225°C/20 mbar gerührt, abgekühlt und mit einer Mischung aus Petrolether (40-60°C) und Benzol aufgenommen. Man erhält 4-(5-Carboxy-2-methylpent-2-yl)phthalsäureanhydrid mit einem Schmelzpunkt 140-143°C.

e) 3,5 Teile dieses Anhydrids und 50 Teile Benzol werden zum Rückfluss erhitzt und während einer Stunde portionsweise 5 Teile Aluminiumtrichlorid zugegeben. Es wird noch 3 Stunden erhitzt und dann mit verdünnter Salzsäure versetzt. Das gebildete Oel wird mit Ether extrahiert, die Lösung mit Wasser gewaschen, der Ether verdampft und der Rückstand in 50 ml 30%-igem Oleum gelöst. Nach 4 Tagen wird die Lösung in 500 Teile Wasser gegossen und mit Ether extrahiert. Der Extrakt wird mit Wasser gewaschen und der Rückstand mit Methanol verestert. Man erhält 5-(2′-Anthrachinoyl)-5-methylhexancarbonsäuremethylester mit einem Siedepunkt von 200°C/0,06 mbar und einem Schmelzpunkt von 70-72°C (umkristallisiert aus Ether/Petrolether). Die Verbindung ist identisch mit der Verbindung gemäss Beispiel 1b.

Beispiel 9: 5-(2′-Anthrachinoyl)-2-methylhexancarbonsäure

a) 5-Methyl-5-phenylhexancarbonsäuremethylester mit einem Siedepunkt von 165-170°C/16 mbar wird durch Umsetzung von 14,2 Teilen 5-Methyl-hex-5-encarbonsäuremethylester, 13,4 Teilen Aluminiumtrichlorid und 39 Teilen Benzol nach dem Verfahren von Beispiel 7a hergestellt.

b) 11 Teile dieser Verbindung, 7,4 Teile Phthalsäureanhydrid, 20,1 Teile Aluminiumtrichlorid und 25 Teile Schwefelkohlenstoff werden gemäss Beispiel 7b umgesetzt und aufgearbeitet. Man erhält 2-Carboxy--4-(5-methoxycarbonyl-2-methylpent-2-yl)-benzophenon mit einem Schmelzpunkt von 138-140° C (Umkristallisation aus Ether).

c) Die alkalische Hydrolyse ergibt 2-Carboxy-4′-(5-carboxy-2-methylpent-2-yl)-benzophenon mit einem Schmelzpunkt von 169-171° C.

d) 0,5 Teile dieser Verbindung werden in 5 Volumenteile 30%-igem Oleum gelöst und 61 Tage stehen gelassen. Man giesst dann in Wasser, filtriert den gelben Feststoff ab, wäscht mit Wasser, trocknet und kristallisiert aus Ether/Petrolether (40-60° C) um. Man erhält 5-(2′-Anthrachinoyl)-2-methylhexancarbonsäure mit einem Schmelzpunkt von 122-124° C, die identisch ist mit der Säure gemäss Beispiel 1c).

Beispiel 10: 5-(2′-Anthrachinoyl)-2-methylhexancarbonsäure

a) 13,6 Teile wasserfreies Zinkchlorid werden in 20,4 Teilen konzentrierter Salzsäure gelöst und mit 39,4 Teilen 2-Aminobenzophenon und 25,6 Teilen 5-Methylhex-5-encarbonsäure versetzt. Die Mischung wird in einem Carius-Gefäss 48 Stunden auf 150° C erhitzt. Man kühlt ab, versetzt mit 500 Teilen Methanol und erhitzt 4 Stunden am Rückfluss. Das überschüssige Methanol wird abgezogen. Der Rückstand wird mit 200 Teilen Ammoniak (0,88 Teil in 200 Teilen Wasser) behandelt. Man extrahiert mit Ether und destilliert. Man erhält eine bei 200-244° C/0,8 mbar siedende Fraktion. Diese Fraktion wird 5 Stunden mit 25 Teilen NaOH in 250 Teilen Wasser am Rückfluss gehalten. Man kühlt ab und trennt das unlösliche Oel ab. Die alkalische Hydrolyselösung wird mit konzentrierter Salzsäure angesäuert und der feste Niederschlag abfiltriert. Man erhält 2-Amino-4-(5-carboxy-2-methylpent-2-yl)-benzophenon mit einem Schmelzpunkt von 101-103° C (Umkristallisation aus Petrolether (40-60° C), der wenig Ether enthält).

b) Diese Säure wird mit Methanol, das mit HCl-Gas gesättigt ist, zu 2-Amino-4-(5-methoxycarbonyl-2-methyl-pent-2-yl)-benzophenon verestert.

Elementaranalyse (Gew.-%):

| | C | H | N |
|---|---|---|---|
| Gef.: | 74,39 | 7,54 | 4,06 |
| Ber.: | 74,31 | 7,42 | 4,13 |

c) 6 Teile dieses Esters werden in 20 Teilen Aceton gelöst, gerührt und auf 5° C gekühlt. Während 1 Stunde werden 10,2 Teile konzentrierte Salzsäure und danach 2,5 Teile Natriumnitrit, gelöst in 5 Teilen Wasser, zugetropft. Es wird noch 1 Stunde nachgerührt und mit 20 Teilen Wasser versetzt. Dann wird die Lösung während 1 Stunde zu einer auf 60° C erwärmten Lösung von 9,2 Teilen CuCN und 9,8 Teilen NaCN in 4 Teilen Wasser getropft. Es wird 2 Stunden bei 75° C weitergerührt. Man extrahiert mit Ether, dampft den Ether ab und chromatographiert an Silica mit Petrolether (40-60° C). Die Elution mit Petrolether und steigenden Mengen (bis 25 Vol.-%) Ether, ergibt 2-Cyano-5-(5-methoxycarbonyl-2-methylpent-2-yl)-benzophenon.

Elementaranalyse (Gew.-%):

| | C | H | N |
|---|---|---|---|
| Gef.: | 75,63 | 6,59 | 3,83 |
| Ber.: | 75,62 | 6,53 | 4,01 |

d) 1,9 Teile dieses Nitrils werden mit 5 Teilen Eisessig, 5 Teilen Wasser und 10 Teilen 98%-iger Schwefelsäure versetzt und 6 Stunden am Rückfluss erhitzt. Man isoliert das Produkt und kristallisiert aus Ethylacetat/Petrolether (40-60° C) um. Man erhält 2-Carboxy-5-(5-carboxy-2-methylpent-2-yl)-benzophenon mit einem Schmelzpunkt von 178-181° C.

e) 0,5 Teile dieser Säure werden in 7,5 Teilen 30%-igem Oleum gelöst und 4 Tage bei Raumtemperatur stehen gelassen. Dann wird die Reaktionsmischung in Wasser gegossen, der Niederschlag abfiltriert, gewaschen, getrocknet und aus Ether umkristallisiert. Man erhält 5-(2′-Anthrachinoyl)-5-Methylhexancarbonsäure mit einem Schmelzpunkt von 123-124° C, die mit der Verbindung von Beispiel 1c identisch ist.

Beispiel 11: 5-(2′-Anthrachinoyl)-5-methylhexancarbonsäure-hydrazid

Eine Mischung von 43,3 g 5-(2′-Anthrachinoyl)-5-methylhexansäuremethylester gemäss Beispiel 1b, 26 ml Hydrazinhydrat und 200 ml Ethanol wird unter Argon während 22 Stunden unter Rückfluss gekocht. Das Gemisch wird auf 400 ml Eiswasser gegossen und mit 500 ml Chloroform extrahiert. Die organische Phase wird mit Wasser gewaschen, getrocknet und eingedampft. Chromatographie des Rückstandes an Kieselgel (1 kg) mit Dichlormethan/Ethanol (40:3) als Eluens gibt 41 g 5-(2′-Anthrachinoyl)-5-methylhexancarbonsäurehydrazid.
Massenspektrum (indirekte Probenzuführung, 205°C): m/e = 350 $M^+$, 100 %).

Beispiel 12: 5-(Anthrachinon-2′-yl)-5-methylhexancarbonsäure-N-1-methylhydrazid

Zu einer Lösung von 6,7 g 5-(Anthrachinon-2′-yl)-5-methylhexancarbonsäure gemäss Beispiel 1c in 30 ml trockenem Tetrahydrofuran (THF) werden bei -15°C unter Argon 2,5 g N-Methylmorpholin und 3,0 g Chlorameisensäure-isobutylester gegeben. Nach 10 Minuten werden 1,85 g N-Methylhydrazin in 20 ml THF zugegeben. Man lässt auf Raumtemperatur erwärmen und rührt 30 Minuten bei dieser Temperatur. Das Tetrahydrofuran wird abgedampft, der Rückstand in Essigester gelöst und mit 5%-iger wässriger Natriumbicarbonat-Lösung gewaschen. Der Rückstand der organischen Phase (8,1 g) wird durch Chromatographie an 1 kg Kieselgel gereinigt. Elution mit Methylenchlorid/Ethylacetat (4/1) gibt 4,8 Hydrazid.
Massenspektrum (indirekte Probenzuführung, 205°C): m/e = 364 (20 %, $M^+$), 46 (100 %).

Elementaranalyse (Gew.-%):

| | C | H | N | O |
|---|---|---|---|---|
| Ber.: | 72,5 | 6,64 | 7,68 | 13,17 |
| Gef.: | 72,0 | 6,7 | 7,4 | 13,7 |

Beispiel 13: 5-(2′-Anthrachinoyl)-5-methylhexancarbonsäure-[N,N-di-(4″-hydroxyphenyl)amid]

a) 5-(2′-Anthrachinoyl)-5-methylhexansäure-chlorid

Zu einer Lösung von 4,06 g 5-(2′-Anthrachinoyl)-5-methylhexancarbonsäure gemäss Beispiel 1c in 20 ml Thionylchlorid werden 0,2 ml N,N-Dimethylformamid gegeben. Es wird 30 Minuten bei Raumtemperatur gerührt und das Reagens bei Normaldruck abdestilliert. Trocknen des Rückstandes am Hochvakuum gibt 5,0 g kristallines Säurechlorid, welches ohne weitere Reinigung weiterverarbeitet wird.

b) 5-(2′-Anthrachinoyl)-5-methylhexancarbonsäure-N,N-bis-(4″-acetoxyphenyl)-amid

Eine Lösung von 1,04 g 5-(2′-Anthrachinoyl)-5-methylhexancarbonsäurechlorid gemäss Beispiel 13a, 558 mg 4,4′-Diacetoxy-diphenylamin [CA 90, 22577d (1979)] und 267 mg p-N,N-Dimethylaminopyridin in 3 ml Pyridin wird 4 Tage bei Raumtemperatur gerührt. Man nimmt in Dichlormethan auf und wäscht mit 2N-Salzsäure und gesättigter Kochsalzlösung. Chromatographie des Rückstandes der organischen Phase an Kieselgel mit Dichlormethan/Toluol/Diethylether (9:9:2) als Eluens gibt 590 mg 5-(2′-Anthrachinoyl)-5-methylhexancarbonsäure-N,N-bis-(4″-acetoxyphenyl)amid. Massenspektrum (indirekte Probenzuführung, 270°C): m/e = 603 ($M^+$, 40 %), 285 (60 %), 243 (90 %), 201 (100 %).

c) 5-(2′-Anthrachinoyl)-5-methylhexancarbonsäure-[N,N-di-(4″-hydroxyphenyl)amid]

Eine Mischung von 358 mg Diacetat gemäss Beispiel 13b, 10ml Methanol, 3 ml Dichlorethan und 0,1 ml Methansulfonsäure wird 45 Minuten unter Rückfluss gekocht. Verdünnen mit Dichlormethan, Waschen mit Wasser und gesättigter Kochsalzlösung, Trocknen und Eindampfen der organischen Phase, sowie Chromatographie des Rückstandes an Keiselgel mit Chloroform/Ethylacetat (3:1) als Eluens gibt 290 mg 5-(2′-Anthrachinoyl)-5-methylhexansäure-[N,N-bis-(4″-hydroxyphenyl)-amid]. Massenspektrum (indirekte Probenzuführung, 320°C): m/e = 519 ($M^+$, 1 %).

B) Anwendungsbeispiele

Beispiel 14:

a) 173,3 g (0,5 mol) Bisphenol-A-diglycidylether wird in 180 ml Ethylenglykolmonoethylether bei 40° C gelöst. Zu dieser Lösung lässt man 15,3 g (0,25 mol) Ethanolamin unter Rühren zutropfen, steigert die Temperatur auf 60° C und lässt noch 3 Stunden reagieren. Man erhält eine viskose Lösung. Das Epoxyequivalent der Lösung beträgt 1,158 mol Epoxid/kg (bestimmt nach der Methode von R.R. Jay, Analytical Chemistry 36 (1964) 667). Dies entspricht einem mittleren Molekulargewicht des Advancementprodukts von 915 Dalton.

5 g der Lösung (5,79 mAequivalent Epoxid) werden mit 2,85 mMol (2-Anthrachinoyl)-5-methylhexancarbonsäurehydrazid unter Rühren und Schutzgas bei 130° C 2 Stunden umgesetzt. Es resultiert eine viskose Lösung mit geringem Epoxidrestgehalt. Die Lösung wird mittels Drahtrakeln als Film auf einen Polyesterträger aufgebracht und im Umluftofen für 12 Stunden bei 80° C getrocknet.

b) Es wird in analoger Weise verfahren, aber in anderen Mengenverhältnissen (siehe Tabelle). In der Tabelle sind die Eigenschaften der Polyaddukte angegeben.

Die Photoeffizienz der Photoreduktion wird wie folgt bestimmt. Ein Film auf einem Polyesterträger mit einer optischen Dichte (O.D.) von 1 bei 324 nm wird mit einer Hg-Hochdrucklampe mit 40 $mW/cm^{-2}$ bestrahlt und in regelmässigen Abständen des UV/VIS-Spektrum aufgenommen. Die Bande bei 324 nm nimmt ab, die Bei 386 nm nimmt zu. Das Verhältnis beider Banden nach 2 Minuten Belichtungszeit wird als Photoeffizienz angenommen.

Tabelle

| Beispiel Nr. | Tg °C | mMol Ausgangsstoffe | | | Gehalt des Adduktes an | | Photoeffizienz |
|---|---|---|---|---|---|---|---|
| | | Verbindung gemäss Bsp. 12 | Ethanolamin | Bisphenol-A-diglycidylether | Anthrachinon (mMol/kg) | OH (mMol/kg) | O.D.324nm / O.D.386nm |
| a | 86 | 1 | 1 | 2 | 0,77 | 3,86 | 0,53 |
| b | 101 | 1 | 2 | 3 | 0,18 | 3,44 | 0,67 |

Photometallisierung

Filme auf einem Polyesterträger werden auf einem thermostatisierbaren Vakuumheiztisch bei 50 °C mit einer HG-Hochdrucklampe mit 40 $mW/cm^{-2}$ Intensität durch ein Negativ belichtet.

Man erhält ein dunkles, negatives Abbild der Vorlage, das in einem Abscheidungsbad der Zusammensetzung:

$CuSO_4 \times 5H_2O$ 0,0665 Mol/l
HCOH 0,0467 Mol/l
Quadrol 0,0599 Mol/l
NaOH pH 12.6
NaCN 25 mg/l
2-Mercaptobenzthiazol 10mg/l
bei 45° C zu einem metallischen Kupferbild verstärkt wird.

**Patentansprüche**

1. Verbindung der Formel I

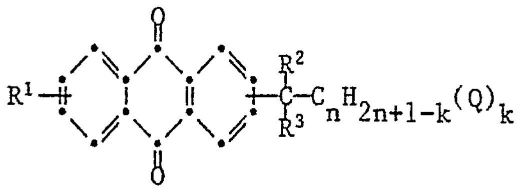 (I) ,

worin
n eine Zahl von 1 bis 17 darstellt,
k für 1 oder 2 steht und Q -COOH oder $-CONR^4-NH_2$ bedeutet, oder k für 1 steht und Q -OH, $-NHR^4$, $-OCOR^5$, $-N(R^4)COR^5$ oder

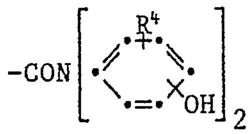

darstellt, worin $R^4$ H oder $C_1$-$C_4$-Alkyl ist und $R^5$ den um eine Carboxylgruppe verminderten Rest einer Tricarbonsäure oder Tetracarbonsäure bedeutet, $R^2$ und $R^3$ unabhängig voneinander lineares oder verzweigtes $C_1$-$C_5$-Alkyl darstellen, das unsubstituiert ist oder durch -COOH oder $-CONR^4-NH_2$ substituiert ist, falls Q -COOH oder $-CONR^4-NH_2$ darstellt, oder $R^2$ zusammen mit der $C_nH_{2n+1-k}$-Gruppe $C_5$-$C_{12}$-Cycloalkylen bildet und $R^3$ für $C_1$-$C_5$-Alkyl steht; und $R^1$ H, lineares oder verzweigtes $C_1$-$C_8$-Alkyl, unsubstituiertes oder mit ein oder zwei Halogen substituiertes $C_7$-$C_9$-Aralkyl, unsubstituiertes oder mit $C_1$-$C_4$-Alkyl substituiertes $C_5$-$C_{12}$-Cycloalkyl bedeutet oder einen Rest der Formel $-CR^2R^3-C_nH_{2n+1-k}(Q)_k$ darstellt.

2. Verbindungen gemäss Anspruch 1, worin in Formel I $R^1$ für H oder einen Rest der Formel $-CR^2R^3C_nH_{2n+1-k}(Q)_k$ steht, $R^2$ und $R^3$ unabhängig voneinander Methyl oder Ethyl bedeuten, n für eine Zahl von 1 bis 8 und k für 1 stehen, Q -COOH oder $-CONR^4-NH_2$ darstellt, und $R^4$ H oder $C_1$-$C_4$-Alkyl bedeutet.

3. Verbindungen gemäss Anspruch 1, worin in Formel I k für 1 steht, n eine Zahl von 1 bis 12 ist und Q die im Anspruch 1 angegebene Bedeutung hat.

4. Verbindungen gamäss Anspruch 1, worin in Formel I $R^1$ für H oder einen Rest der Formel $-CR^2R^3-C_nH_{2n+1-k}(Q)_k$ stehen.

5. Verbindungen gemäss Anspruch 1, worin in Formel I der Rest $-CR^2R^2C_nH_{2n+1-k}(Q)_k$ in 2-Stellung und der Rest $R^1$ in 6- oder 7-Stellung gebunden sind.

6. Verbindungen gemäss Anspruch 5, worin $R^1$ $-CR^2R^3C_nH_{2n+1-k}(Q)_k$ ist.

7. Verbindungen gemäss Anspruch 1, worin Q $-CONR^4-NH_2$ bedeutet und $R^4$ für H oder Methyl steht.

8. Verbindungen der Formel Ia

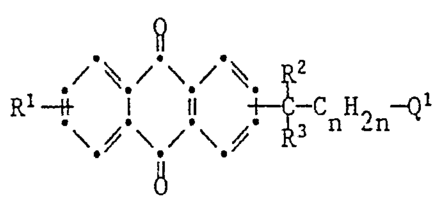 (Ia)

worin
$R^1$ H, lineares oder verzweigtes $C_1$-$C_8$-Alkyl, unsubstituiertes oder mit 1 oder 2 Halogen substituiertes $C_7$-$C_9$-Aralkyl, unsubstituiertes oder mit $C_1$-$C_4$-Alkyl substituiertes $C_5$-$C_{12}$-Cycloalkyl oder einen Rest der Formel $-CR^2R^3-C_nH_{2n}-Q^1$ darstellt; $R^2$ und $R^3$ unabhängig voneinander lineares oder verzweigtes $C_1$-$C_5$-Alkyl darstellen, oder $R^2$ zusammen mit der $C_nH_{2n}$-Gruppe $C_5$-$C_{12}$-Cycloalkylen bildet und $R^3$ H oder $C_1$-$C_5$-Alkyl ist; n für eine Zahl von 1 bis 17 steht; und $Q^1$ für -CN, -COCl, $-CONHR^4$, $-CON(R^4)_2$, $-COR^6$, -O-$C_1$-$C_8$-Acyl oder $-NR^4$-$C_1$-$C_8$-Acyl steht, worin $R^4$ H oder $C_1$-$C_4$-Alkyl bedeutet und $R^6$ der Rest eines einwertigen Alkohols ist.

9. Verfahren zur Herstellung von Verbindungen der Formeln I oder Ia gemäss den Ansprüchen 1 und 8, dadurch gekennzeichnet, dass man

a) ein Anthracen der Formel II

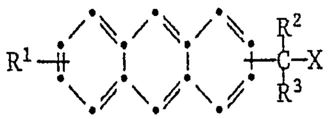

(I)

worin X $-C_nH_{2n+1-k}(Q)_k$ oder $-C_nH_{2n}-Q^1$ ist und $R^1$, $R^2$, $R^3$, Q, $Q^1$, n und k die angegebene Bedeutung haben, zu einer Verbindung der Formel I oder Ia oxidiert, oder

b) ein substituiertes Benzol der Formel III bzw. IIIa

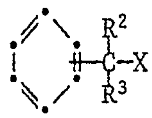

(III)

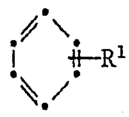

(IIIa)

mit einem Phthalsäureanhydrid der Formeln IV oder IVa

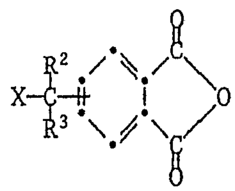

(IV),

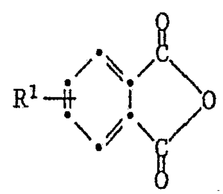

(IVa)

zu Benzophenonverbindungen der Formeln V, Va oder Vb

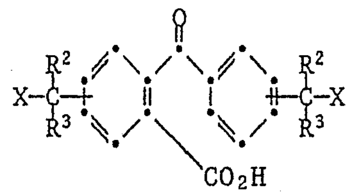

(V),

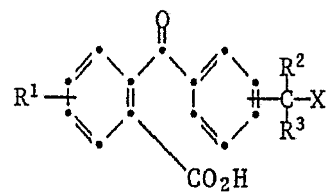

(Va),

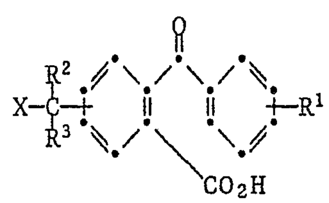

(Vb)

umsetzt, und die Benzophenonverbindungen anschliessend zu den Verbindungen der Formeln I oder Ia cyclisiert.

10. Härtbare Zusammensetzung, enthaltend

a) mindestens ein Epoxidharz mit durchschnittlich mehr als einer Epoxidgruppe im Molekül,

b) gegebenenfalls einen Härter für das Epoxidharz,

c) ein Anthrachinon der Formel I gemäss Anspruch 1 oder Mischungen hiervon und

d) mindestens ein primäres oder sekundäres aliphatisches Amin, das im aliphatischen Rest mindestens eine Hydroxylgruppe enthält.

11. Zusammensetzung gemäss Anspruch 10, worin
    c) das Anthrachinon der Formel I in einer Menge von 0,1-1 Mol/kg Epoxidharz und
    d) das Amin in einer Menge von 0,1-1,2 Mol/kg Epoxidharz enthalten sind.

12. Zusammensetzung gemäss Anspruch 10, worin als Epoxidharz glycidylisierte Novolake, Hydantoine, Aminophenole, Bisphenole oder aromatische Diamine enthalten sind.

13. Zusammensetzung gemäss Anspruch 12, worin das Epoxidharz ein glycidylisierter Kresolnovolak, Bishphenol-A-glycidylether, mit Bisphenol-A vorverlängerter Bisphenol-A-diglycidylether, Hydantoin-N,N'-bisglycid, Propylen-1,3-bis-hydantoin-2-hydroxy-triglycid, p-Aminophenoltriglycid, Diaminodiphenylmethan-tetraglycid oder Mischungen hiervon ist.

14. Zusammensetzung gemäss Anspruch 10, worin der Härter ein Novolak, ein Polyamin oder ein Polycarbonsäureanhydrid ist.

15. Zusammensetzung gemäss Anspruch 10, worin im Anthrachinon der Formel I $R^1$ für H steht, $R^2$ und $R^3$ unabhängig voneinander Methyl oder Ethyl darstellen, $k = 1$ und n eine Zahl von 1 bis 5 sind, und Q $-CO-NR^4-NH_2$ bedeutet, worin $R^4$ für H oder Methyl steht.

16. Zusammensetzung gemäss Anspruch 15, dadurch gekennzeichnet, dass es sich bei dem Anthrachinon der Formel I um ein solches der Formel

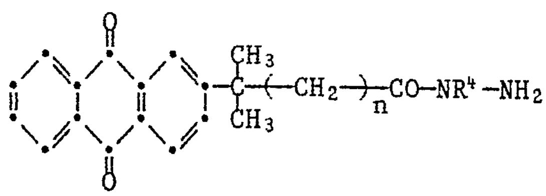

handelt, worin n eine Zahl von 1 bis 3 und $R^4$ H oder Methyl sind.

17. Zusammensetzung gemäss Anspruch 10, worin das Amin der Komponente d) der Formel VI

$$H-N(R^7)-C_yH_{2y+1-x}(OH)_x \qquad (VI),$$

entspricht,
worin $R^7$ H, lineares oder verzweigtes $C_1$-$C_{18}$-Alkyl, $C_3$-$C_7$-Cycloalkyl, $C_6$-$C_{10}$-Aryl, $C_7$-$C_{18}$-Alkaryl, $C_7$-$C_{12}$-Aralkyl, $C_8$-$C_{18}$-Alkaralkyl oder die Gruppe $-C_yH_{2y+1-x}OH_x$ bedeutet, x für eine Zahl von 1 bis 3 und y für eine Zahl von 2 bis 12 stehen, wobei die Gruppe $C_yH_{2y}$ durch ein oder mehrere -O- oder $-NR^7$-unterbrochen sein kann.

18. Zusammensetzung gemäss Anspruch 17, worin in Formel VI $R^7$ für H steht, y eine Zahl von 2 bis 7 und x eine Zahl von 1 bis 3 bedeuten.

19. Gehärtete Zusammensetzung aus
    a) mindestens einem Epoxidharz mit durchschnittlich mehr als einer Epoxidgruppe im Molekül,
    b) gegebenenfalls einem Härter für das Epoxidharz,
    c) einem Anthrachinon der Formel I gemäss Anspruch 1 oder Mischungen hiervon und
    d) mindestens einem primären oder sekundären aliphatischen Amin, das im aliphatischen Rest mindestens eine Hydroxylgruppe enthält.

20. Verwendung einer gehärteten Zusammensetzung gemäss Anspruch 10 zur Herstellung von metallischen Ueberzügen oder Abbildungen durch stromlose Metallabscheidung nach einer vollständigen oder teilweisen Belichtung der Oberfläche.